# EUROPEAN PATENT SPECIFICATION

(11) **EP 4 081 115 B1**
(45) Date of publication and mention of the grant of the patent: **21.10.2026**
(21) Application number: 20905911.2
(22) Date of filing: 18.12.2020
(51) Int. Cl.: A61B 5/103, A61B 5/00, A61B 5/107, A61B 18/20

(54) **SYSTEMS, METHODS AND COMPUTER-ACCESSIBLE MEDIUM FOR PROVIDING FEEDBACK AND ANALYSIS ON AN ELECTROMAGNETIC-BASED TREATMENT DEVICE**
SYSTEME, VERFAHREN UND RECHNERGESTEUERTES MEDIUM ZUM FEEDBACK UND ANALYSIEREN AUF EINER ELEKTROMAGNETISCHEN BEHANDLUNGSVORRICHTUNG
SYSTÈMES, PROCÉDÉS ET SUPPORT ACCESSIBLE PAR ORDINATEUR POUR FOURNIR UNE RÉTROACTION ET UNE ANALYSE SUR UN DISPOSITIF DE TRAITEMENT À BASE ÉLECTROMAGNÉTIQUE

(30) Priority: 23.12.2019 US 201962952694 P
(43) Date of publication of application: 02.11.2022
(73) Proprietor: Blossoms Innovations, LLC, Boston, Massachusetts 02114 (US)
(72) Inventor: ZUO, Vincent, Boston, Massachusetts 02114 (US); LEI, Hao, Waltham, Massachusetts 02453 (US); DODD, Thomas, Upton, Massachusetts 01568 (US); BHAWALKAR, Jayant, Auburndale, Massachusetts 02466 (US); LEVINE, Lewis J., Marlborough, Massachusetts 01752 (US); DRESSER, Charles Holland, Wayland, Massachusetts 01778 (US); KATKAM, Rajender, Waltham, Massachusetts 02452 (US)
(74) Representative: LKGLOBAL Partnerschaftsgesellschaft mbB
(86) International application number: PCT/US2020/066032
(87) International publication number: WO 2021/133676

(56) References cited:
- EP-A1- 3 446 750
- WO-A1-2018/017811
- WO-A1-2018/152538
- DE-A1- 102017 116 004
- KR-A- 20090 059 667
- US-A1- 2003 065 278
- US-A1- 2008 033 410
- US-A1- 2008 247 637
- US-A1- 2009 149 843
- US-A1- 2009 149 843
- US-A1- 2014 111 621
- US-A1- 2016 307 057
- US-A1- 2019 274 759

## Description

### FIELD OF THE DISCLOSURE

The present disclosure relates to feedback and analysis of an electromagnetic-based treatment device as well as treatment of skin of a subject, and more particularly to Systems, non-therapeutic and non-surgical methods and computer-accessible medium for providing feedback and analysis of such electromagnetic-based treatment device, and the treatment of skin using such exemplary Systems, method and computer-accessible medium.

### BACKGROUND INFORMATION

Cosmetic and dermatological treatments commonly use energy-based devices (e.g., electromagnetic radiation (EMR), lasers, etc.). Typically, energy can be selectively delivered to tissue based upon a wavelength of the EMR. Targets within the tissue (e.g., chromophores) likely absorb certain wavelengths, such that energy can be delivered selectively to the target tissue, and is not absorbed by the untargeted tissue. While this method of treatment has been successful for a number of treatments and populations of patients, there exist certain populations who remain underserved by prior energy-based treatments.

Wavelength selectivity has been recently used for many energy-based treatments, including hair removal and tattoo removal. Selectively targeting a Chromophore within a tissue has been used for a number of years. However, in various cases, wavelength selectivity has been poor, and treatment results have suffered.

For example, recently, laser hair removal has been performed with a laser that is absorbed by pigment (e.g., melanin) within the hair follicle (e.g., in dark hair). This technique only works for patients whose hair contains pigment (i.e., is brown), and whose skin does not contain pigment (i.e., being white). Currently, patients who have darker skin or lighter hair (e.g., blonde or white) may not effectively employ the existing laser hair removal treatments.

US 2008/0033410 A1 describes a method for automated treatment of an area of skin of a patient with laser energy. The method including: identifying the area to be treated with the laser; modeling the identified area of the skin to be treated; and controlling the laser to direct laser energy to within the modeled area of the skin.

WO 2018/017811 A1 describes automated systems and methods for removing hair from a body surface. The methods and systems are semi- or fully automated. Some of the steps of the methods comprise determining a hair growth phase and selecting hair follicles for depilation based at least in part of the identified hair growth phase. The energy delivery device may be attached to the robotic arm and directed to align with the hair follicle to be depilated.

WO 2018/152538 A1 describes an automated laser-surgery system for performing a closed-loop surgical procedure. The procedure includes forming a post-procedural goal based on a three-dimensional ( 3D) image of a surgical site, planning a path for a surgical laser signal based on the post-procedural goal, performing a procedural pass by steering the surgical laser signal along the path, measuring the surface of the surgical site after the procedural pass, updating a model based on the measured effect at the surgical site, and evaluating the success of the procedural pass based on the surface measurement and the post-procedural goal. If necessary, a new path is planned based on the post-procedural goal and the surface measurement a new pass based on that path is performed, and the surface is again measured to evaluate the success of the new pass. These operations are repeated as a closed-loop sequence as many times as necessary to achieve success.

US 2008/0247637 A1 describes a robotic tattoo application and tattoo removal methods and systems. This technology involves the use of a robotic system guided by control of a graphics capable computer in order to perform various types, including artistic, recreational, cosmetic, or therapeutic tattooing, or tattoo removal.

### OBJECTS OF THE PRESENT DISCLOSURE

It can be important for such patients to effectively utilize a laser hair removal for laser selectivity that delivers the laser energy to the target tissue (e.g., follicle) without relying on selective absorption at the target site.

To address such deficiencies and issues, it is beneficial to provide spatially selective EMR treatments, as described herein, that use feedback (e.g., images) from the tissue to direct EMR to specific regions for treatment.

Further one of the objects of the present disclosure is to provide treatment that can effectuate and/or alter subject's outward appearance that is pleasing to the subject. Any number of difficulties can occur when attempting to reach a goal that is not quantifiable. However, to assist with such goal, it is possible to take images of the subject, which can capture the subject's appearance at the time of visit.

Thus, there may be a need to address at least some of the deficiencies described herein above, with the exemplary embodiments of the present disclosure as discussed herein below.

### SUMMARY OF EXEMPLARY EMBODIMENTS

Such needs are addressed by the subject-matter of each of the independent claims. Further embodiments according to the invention are defined by the dependent claims. Exemplary Systems, methods and computer-accessible medium can be provided that can be configured to capture images of the subject (and/or portions thereof), under the same conditions, and record the images for later viewing, analysis, and comparison, according to certain exemplary embodiments of the present disclosure. Although the invention is in principle suitable i. a. also for use in surgical and therapeutic methods, such as treatment of acne and resurfacing scar tissue mentioned hereinafter, therapeutic and surgical methods do not form part of the present invention.

The invention defined in the set of claims is suitable for performing a feedback-controlled electromagnetic radiation (EMR)-based treatment.

The system includes a detector configured to detect a feedback (e.g., a first feedback) of a tissue, a Computer storage device configured to record the feedback thereon or therein, an EMR source configured to generate an EMR beam, and preferably an optical arrangement configured to direct the EMR beam toward the tissue. A Controller can be provided configured to (i) recognize one or more targets within the portion(s) of the tissue based upon the feedback data, (ii) locate one or more Coordinates within the portion(s) associated with the target(s), and (iii) control the optical arrangement to direct the EMR beam to impact the coordinate(s).

The EMR beam is absorbable by the tissue, and can have a wavelength within a set of ranges which are, e.g., (i) 200 - 500nm, (ii) 1300 - 3500nm, and/or (iii) 9-1 Ipm. The target(s) can comprise (i) a sebaceous gland, (ii) a eccrine gland, (iii) a hair follicle, and/or (iv) a tattoo.

In some exemplary embodiments of the present disclosure, the characteristic(s) of the EMR beam can comprise a scan pattern, a pulse duration, a pulse energy, a repetition rate, and/or a wavelength. In some exemplary embodiments, the feedback can comprise an image, and the Controller can comprise an image recognition configuration. According to various exemplary embodiments of the present disclosure, the image recognition configuration can comprise an edge detection module, a comer detection module, and/or a blob detection.

The detector is further configured to detect another feedback (e.g., a second feedback) of the tissue. The Controller is further configured to compare the first feedback and the second feedback, and determine - based on the comparison - a suggested course of treatment, a probable diagnosis, a characteristic related to treatment progression, and/or a characteristic of the tissue. In some exemplary versions, the Controller can further comprise a neural network, an artificial intelligence, a clinical decision Support System, and/or a machine vision. According to some exemplary versions, the detector can be configured to allow a time, e.g., longer than 12 hours, to elapse between detecting the first feedback and the second feedback. According to some exemplary embodiments of the present disclosure, the Computer storage device can include a network storage device, a hard disk drive, a memory device and/or an electronic health record.

In some exemplary embodiments of the present disclosure, the detector can include a camera, an ultrasound transducer, a photoacoustic imaging System, an optical coherence tomography System, an optical coherence elastography System, a coherent anti-stokes Raman spectroscopy imaging System, a two-photon imaging System, second harmonic generation imaging System, a phase conjugate imaging System, a hyperspectral imaging System, a low-power carbon-dioxide laser imaging System, X-ray backscatter imaging System, a millimeter wave imaging System, a magnetic resonance imaging System, a high-frequency ultrasound imaging System, a photodiode, an ultrasound transducer array, a fluoroscope, a surface profilometer, an infrared imaging System, and/or a confocal microscope.

According to certain exemplary embodiments of the present disclosure, the exemplary System can further comprise a structured light source, and the Controller can include a fringe projection profilometry configuration, a structure light profilometry configuration, a laser triangulation profilometry configuration, and/or a Stereovision measurement configuration. According to certain other exemplary embodiments of the present disclosure, a method can be provided for performing a feedback-controlled electromagnetic radiation (EMR)-based treatment. Such exemplary method can comprise detecting, using a detector, a feedback (e.g., a first feedback) of a tissue, storing the feedback to a Computer storage device, generating, using an electromagnetic radiation (EMR) source, an EMR beam, recognizing, using a Controller, one or more targets within the portion(s) of the tissue based upon the feedback data, locating, using the Controller, one or more Coordinates within the portion(s) associated with the target(s), and Controlling, using the Controller, the optical arrangement to direct the EMR beam to impact the coordinate(s).

According to certain exemplary embodiments of the present disclosure, the EMR beam can be absorbable by the tissue, and can have a wavelength within a set of ranges which are, e.g., (i) 200 - 500nm, (ii) 1300 - 3500nm, and/or (iii) 9-1 Ipm. The target(s) can comprise (i) a sebaceous gland, (ii) a eccrine gland, (iii) a hair follicle, and/or (iv) a tattoo.

In some exemplary embodiments of the present disclosure, exemplary characteristic(s) of the EMR beam can include a scan pattern, a pulse duration, a pulse energy, a repetition rate, and/or a wavelength. In some exemplary versions, the first feedback can comprise an image. Further, it is possible to perform the image recognition based on the first feedback. In some exemplary cases, the performance of the image recognition can include performing edge detection, comer detection, and/or blob detection.

In some exemplary embodiments of the present disclosure, the exemplary method can additionally include detecting, using a detector, a further (e.g., second) feedback of the tissue, comparing the (first) feedback and the further/second feedback, and determining from the comparison a suggested course of treatment, a probable diagnosis, a characteristic related to treatment progression, and a characteristic of the tissue. In some exemplary versions, determining from the comparison is done using at least one of a neural network, artificial intelligence, clinical decision Support, and/or machine vision. In some exemplary versions, a time, longer than 12 hours, lapses between detecting the (first) feedback and the further/second feedback.

In some exemplary embodiments of the method, the Computer storage device can include a network storage device, a hard disk drive, a memory device, and/or an electronic health record.

In some exemplary embodiments of the present disclosure, the detector can include a camera, an ultrasound transducer, a photoacoustic imaging System, an optical coherence tomography System, an optical coherence elastography System, a coherent anti-stokes Raman spectroscopy imaging System, a two-photon imaging System, second harmonic generation imaging System, a phase conjugate imaging System, a hyperspectral imaging System, a low-power carbon-dioxide laser imaging System, X-ray backscatter imaging System, a millimeter wave imaging System, a magnetic resonance imaging System, a high-frequency ultrasound imaging System, a photodiode, an ultrasound transducer array, a fluoroscope, a surface profilometer, an infrared imaging System, and/or a confocal microscope.

In some exemplary embodiments of the present disclosure, the method can additionally include directing a structured light to the tissue; and the detection of the (first) feedback can include performing fringe projection profilometry, structure light profilometry, laser triangulation profilometry, and/or Stereovision measurement.

According to another exemplary embodiment of the present disclosure, a System can be provided for feedback-controlled removal of a tattoo which can comprise a sensor configured to capture an image (e.g., a first image) of the tattoo, a Controller configured to identify a plurality of non-adjacent locations of the tattoo from the (first) image, a laser source (or electromagnetic radiation source EMR) configured to generate a laser beam (or an EMR beam), and a laser beam (or EMR) scanning System registered to the image, and configured to deliver the laser beam (or the EMR beam) to the non-adjacent location(s) of the tattoo.

In some exemplary embodiments of the present disclosure, the exemplary System can be additionally configured to capture another (second) image of the tattoo after delivering the laser beam to the location of the tattoo; and the System can further comprise a Computer storage device (e.g., a memory) configured to store at least one of the (first) image and the further/second image. In some exemplary versions, the sensor can be additionally configured to capture a further (third) image of the tattoo after the laser treatment. The Controller can be further configured to identify a plurality of further non-adjacent locations of the tattoo from the further Information; and wherein the beam scanning System is controlled by the Controller to deliver the EMR beam to the plurality of further non-adjacent locations of the tattoo.

In some exemplary versions, the Computer storage device can comprise a network storage device, a hard disk drive, a memory device and/or an electronic health record.

In some exemplary embodiments of the present disclosure, the exemplary Controller can further include an image recognition System configured to recognize the tattoo within the (first) image. In some exemplary versions, the image recognition System can include edge detection, feature detection, Canny edge detection, Sobel edge detection, Shi and Tomasi comer detection, features from accelerated segment test (FAST) comer and blob detection, histogram of oriented gradients (HOG), scale-invariant feature transform (SIFT), speeded up robust feature (SURF), and/or thresholding for edge detection. In some exemplary versions, the sensor can include two or more cameras, and the image recognition System can include a Stereovision measurement. In some exemplary versions, the exemplary System can also include a structured light source, and the image recognition System can include fringe projection profilometry, structure light profilometry, laser triangulation, and/or Stereovision measurement.

In some exemplary embodiments of the present disclosure, the exemplary sensor can comprise a photodiode.

A further exemplar embodiment of a method for feedback-controlled removal of a tattoo can be provided which can comprise identifying, using the Controller, a plurality of further non-adjacent locations of the tattoo from the Information and the further Information, and directing, using the laser beam scanning System controlled by the Controller, the laser beam to the plurality of further non-adjacent locations of the tattoo.

In some exemplary embodiments of the present disclosure, the exemplary method can also include capturing, using the sensor, another (second) image of the tattoo after delivering the laser beam to the location of the tattoo; and, storing in and/or to a Computer storage arrangement (e.g., memory) the (first) image and the other (second) image. In some exemplary versions, the exemplary method can also include capturing, using the sensor, a further (e.g., third) image of the tattoo after treatment, reading from the Computer storage arrangement the (first) image and the other (second) image, identifying, using the Controller, a new location of the tattoo from the further (third) image and the (first) image and the other (second) image. In some exemplary versions, the Computer storage device can include a network storage device, a hard disk drive, a memory device and/or an electronic health record.

In some exemplary embodiments of the present disclosure, the identification of the location of the tattoo from the (first) image can include recognizing, using an image recognition System, the tattoo within the (first) image. In some exemplary versions, the exemplary image recognition System can include edge detection, feature detection, Canny edge detection, Sobel edge detection, Shi and Tomasi comer detection, features from accelerated segment test (FAST) comer and blob detection, histogram of oriented gradients (HOG), scale-invariant feature transform (SIFT), speeded up robust feature (SURF), and/or thresholding for edge detection. In some exemplary versions, the exemplary sensor can include two or more cameras, and recognizing the tattoo can include a stereoscopic measurement. In some exemplary versions, the exemplary method can also include directing a structured light to the tissue, and recognizing the tattoo can include fringe projection profilometry, structure light profilometry, laser triangulation profilometry, and/or Stereovision measurement. In some exemplary versions, the identification of the location of the tattoo can comprise performing a homography transform, an affine transform, image registration, and/or Stereovision measurement.

In some exemplary embodiments of the present disclosure, the sensor can include a photodiode.

According to further exemplary embodiments of the present disclosure, an exemplary System can be provided that can comprise (i) an image acquisition device configured to obtain an image of a tattoo on a tissue, (ii) a Controller configured to identify a plurality of non-adjacent locations of the tattoo based on Information associated with the image, (iii) an electromagnetic radiation (EMR) source configured to generate an EMR beam, and (iv) a beam scanning System which is registered to the image, and controlled by the Controller and configured to direct and deliver the EMR beam to impact the non-adjacent locations of the tattoo.

For example, the image acquisition device can be further configured to obtain a further image of the tattoo aller delivering the EMR beam to the non-adjacent locations of the tattoo. Such image acquisition device can further comprise a digital storage device configured to store the Information and/or a further Information which can be based on the further image. The Controller can be further configured to identify a plurality of further non-adjacent locations of the tattoo from such further Information. The beam scanning System can be controlled by the Controller to deliver the EMR beam to such further non-adjacent locations of the tattoo. The digital storage device can comprise a network storage device, a flash drive, a USB drive, a hard disk drive, and/or a memory device, and the digital storage device can be configured to store an electronic health record.

In one exemplary embodiment, the exemplary Controller can further comprise an image recognition System configured to recognize the tattoo within the image. The image recognition System can comprise at least one module which can perform edge detection, feature detection, Canny edge detection, Sobel edge detection, Shi and Tomasi comer detection, features from accelerated segment test (FAST) comer and blob detection, histogram of oriented gradients (HOG), scale-invariant feature transform (SIFT), speeded up robust feature (SURF), and/or thresholding for edge detection. Alternatively or in addition, the image acquisition device can comprise two or more Cameras, and the image recognition System can comprise a Stereovision measurement configuration. The exemplary System can further comprise a structured light source, and the image recognition System can comprise a fringe projection profilometry configuration, a structure light profilometry configuration, a laser triangulation profilometry configuration, and/or a Stereovision measurement configuration. The Controller can be further configured to perform a homography transform procedure, an affine transform procedure, an image registration procedure, and/or a Stereovision measurement procedure.

In yet another exemplary of the present disclosure, the image acquisition device can comprise a photodiode. Further or alternatively, the EMR beam can be absorbable by the tissue, and can have a wavelength within a set of ranges which are (i) 200 - 500nm, (ii) 1300 - 3500nm, and/or (iii) 9 - 11pm.

According to still a further exemplary embodiment of the present disclosure, an exemplary method can be provided which can comprise, e.g., (i) obtaining, using an image acquisition device, an image of a tattoo on a tissue, (ii) identifying, using a Controller, a plurality of non-adjacent locations of the tattoo based on Information associated with the image, (iii) generating, using an electromagnetic radiation (EMR) source, an EMR beam, (iv) delivering, using an EMR beam scanning System that is registered to the image, and (v) Controlling, by the Controller, the EMR beam to impact the non-adjacent locations of the tattoo.

In yet a further exemplary embodiments of the present disclosure, the exemplary method can further comprise, e.g., (a) capturing, using the image acquisition device, a further image of the tattoo after delivering the EMR beam to the non-adjacent locations of the tattoo, and (b) storing to a digital storage device at least one of the Information or a further Information which is based on the further image. The exemplary method can further comprise (c) identifying, using the Controller, a plurality of further non-adjacent locations of the tattoo from the Information and/or the further Information, and (d) directing, using the EMR beam scanning System controlled by the Controller, the EMR beam to such further non-adjacent locations of the tattoo. For example, the digital storage device can comprise a network storage device, a flash drive, a USB drive, a hard disk drive, and/or a memory device, and the digital storage device can be configured to store an electronic health record.

In an additional exemplary embodiment of the present disclosure, the identifying of the non-adjacent locations of the tattoo can comprise recognizing, using an image recognition System, the tattoo within the image. The image recognition System can comprise at least one module which can perform edge detection, feature detection, Canny edge detection, Sobel edge detection, Shi and Tomasi comer detection, features from accelerated segment test (FAST) comer and blob detection, histogram of oriented gradients (HOG), scale-invariant feature transform (SIFT), speeded up robust feature (SURF), and/or thresholding for edge detection. The image acquisition device can comprise two or more cameras, and the recognition of the tattoo can comprise a Stereovision measurement.

Additionally, e.g., the exemplary method can further comprise directing a structured light to the tissue, and the recognition of the tattoo can comprise performing fringe projection profilometry, structure light profilometry, laser triangulation profilometry, and/or Stereovision measurement. In addition or alternatively, the Identification of the non-adjacent locations of the tattoo can comprise performing a homography transform, an affine transform, image registration, and/or Stereovision measurement.

According to still further exemplary embodiments of the present disclosure, various computer-accessible medium having Computer Software thereon can be provided, whereas, when the Computer Software is executed by a Computer processor, the Computer processor is configured to perform all of the various exemplary procedures and methods described herein.

These and other objects, features and advantages of the exemplary embodiments of the present disclosure will become apparent upon reading the following detailed description of the exemplary embodiments of the present disclosure, when taken in conjunction with the appended Claims.

### BRIEF DESCRIPTION OF THE DRAWINGS

Further objects, features and advantages of the present disclosure will become apparent from the following detailed description taken in conjunction with the accompanying Figures showing illustrative embodiments of the present disclosure, in which:
FIG. 1 is a diagram of an apparatus for an electromagnetic radiation (EMR) treatment, feedback detection, and/or analysis, according to some exemplary embodiments of the present disclosure;
FIG. 2A is a flow diagram of a method for the EMR treatment, feedback detection, and/or analysis, according to some exemplary embodiments of the present disclosure;
FIG. 2B is a diagram a data storage, according to some exemplary embodiments of the present disclosure;
FIG. 3 is diagram of a feedback directed fractionated tattoo removal apparatus and an exemplary interaction between components thereof, according to some exemplary embodiments of the present disclosure;
FIG. 4 is a flow diagram for the feedback directed fractionated tattoo removal method, according to some exemplary embodiments;
FIG. 5A is an Illustration of an exemplary progression of the feedback directed fractionated tattoo removal treatment, according to some exemplary embodiments of the present disclosure;
FIG. 5B is an Illustration of an exemplary progression of the feedback directed fractionated tattoo removal treatment with untreated fiducials, according to some exemplary embodiments of the present disclosure;
FIG. 6 is an Illustration of a cross section of an exemplary skin tissue portion being impacted by the exemplary target selective feedback-controlled treatment, according to some exemplary embodiments;
FIG. 7A is an Illustration of an exemplary scar tissue texturing pattern for the feedback-controlled scar tissue resurfacing treatment, according to some exemplary embodiments of the present disclosure;
FIG. 7B is an Illustration of an exemplary feedback directed scar tissue resurfacing procedure, according to some exemplary embodiments of the present disclosure;
FIG. 7C is an Illustration of the exemplary fractionated feedback directed scar tissue resurfacing procedure, according to some exemplary embodiments of the present disclosure;
FIG. 8A is a photograph showing an exemplary System performing object recognition, according to some exemplary embodiments of the present disclosure;
FIG. 8B is a photograph showing the exemplary System selectively targeting a region for electromagnetic radiation, according to some exemplary embodiments of the present disclosure; and
FIG. 8C is a photograph showing a region after being selectively targeted by an electromagnetic radiation, according to some exemplary embodiments of the present disclosure.

It is noted that the drawings are not necessarily to scale. The drawings are intended to depict only typical aspects of the subject matter disclosed herein, and therefore should not be considered as limiting the scope of the disclosure. The Systems, devices, and methods specifically described herein and illustrated in the accompanying drawings are non-limiting exemplary embodiments and the appended Claims.

### DETAILED DESCRIPTION OF EXEMPLARY EMBODIMENTS

Certain exemplary embodiments will now be described to provide an Overall understanding of the principles of the structure, function, manufacture, and use of the devices and methods disclosed herein. One or more examples of these exemplary embodiments are illustrated in the accompanying drawings. Those skilled in the art will understand that the exemplary Systems, devices and methods specifically described herein and illustrated in the accompanying drawings are non-limiting exemplary embodiments and that the scope of the present disclosure is defined solely by the Claims (which can be amended or modified as applicable). The exemplary features illustrated or described in connection with one exemplary embodiment can be combined with the features of other exemplary embodiments. Such exemplary modifications and variations are intended to be included within the scope of the present disclosure.

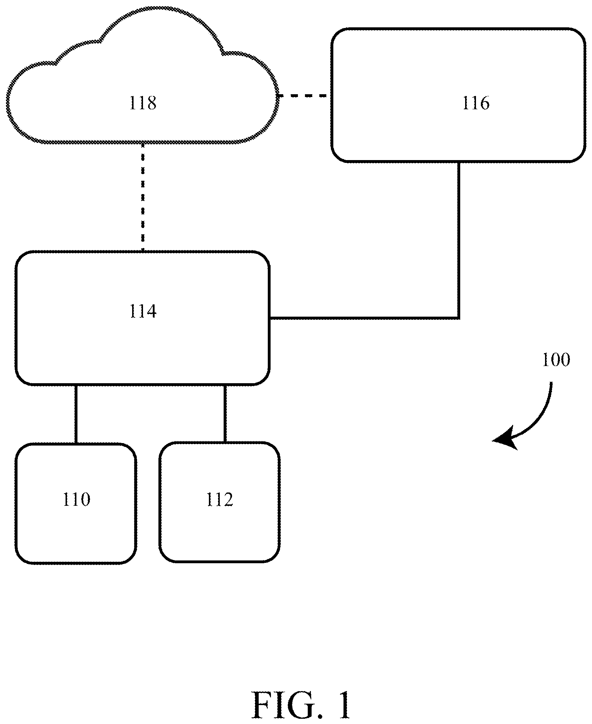

FIG. 1 shows a block diagram of a System 100 is described for electromagnetic radiation (EMR)-based treatment, feedback detection, and analysis according to exemplary embodiments of the present disclosure. The exemplary System 100 can include an exemplary EMR treatment Subsystem 110 that can be configured to perform EMR-based treatment(s) on tissue. Exemplary EMR-based treatment(s) can include and not limited to, e.g., scar resurfacing, bum resurfacing, selective sebaceous gland disruption, skin rejuvenation, selective eccrine (e.g., sweat) gland disruption, pigmented lesion removal, cellulite (striae) remodeling, vascular anomalies (e.g., Parkes Weber syndrome [PWS] and rosacea), hair removal regardless of color and growth phase (i.e. single treatment), Actinic Keratosis, Seborrheic Keratosis, Basal Cell Carcinoma, Port Wine stains, and tattoo removal. In some exemplary embodiments, the EMR treatment Subsystem 110 can comprise (i) a laser source which can be configured to generate a laser beam, and (b) at least one optical arrangement which can be configured to direct the laser beam to the tissue. In various exemplary embodiments, the exemplary treatment System 110 can additional comprise a beam Scanner subsystem(s) which can include galvanometers, spinning mirrors, Risley prisms, and translating optical stages. The exemplary System 100 can include a feedback Subsystem 112 which can be configured to detect feedback from the tissue. Examples of feedbacks from the tissue can include images of the tissue and digitized analog signals from sensors (e.g., cameras, photodiodes, thermocouples, thermistors, etc.). For example, the feedback Subsystem 112 can comprises a detector configured to detect feedback from the tissue. An exemplary feedback System can comprise a structured light source and a camera configured for structured light profilometry.

Both the EMR treatment Subsystem 110 and the feedback Subsystem 112 can communicate with a Controller 114. According to some exemplary embodiments, the Controller 114 can be configured to control one or more parameters of the EMR treatment Subsystem 110 based upon the feedback from the feedback Subsystem 112. For example, the Controller 114 can control a scan pattern, a pulse duration, a pulse energy, a repetition rate, and/or a wavelength of the radiation. The Controller 114 can also communicate (e.g., store the feedback) with a data storage Subsystem 116, which can comprise one or more non-volatile memories, solid state memories (e.g., memory cards), hard disk drives, and/or cloud storage. In some cases, an exemplary picturing archiving and communication System (PACS) can be utilized for physical storage, and digital imaging and Communications in Medicine (DICOM) System can be used as a data format for the feedback. DICOM is a Standard maintained by Health Level Seven (HL7) Standards group. Data associated with the feedback in some exemplary embodiments of the present disclosure can be moved into and out of the cloud. Data exchange with the remote data storage in some cases can be performed a fast healthcare interoperability resources (FHIR) Service, currently being implemented by numerous vendors, for example, including Microsoft Azure cloud Service and Google's Cloud Healthcare Service.

In some exemplary embodiments (e.g., cloud Computing), the Controller 114 can communicate with the data storage Subsystem 116 via one or more networks 118. Exemplary networks 118 as define by topology can include one or more of local area networks (LAN), Controller area networks (CAN), wide area networks (WAN), and/or wireless local area networks (e.g., Wi-Fi). Networks can be based on communication protocols such as, e.g., TCP/IP, GSM, CDMA, etc. and established over a variety of media such as wired, wireless, Bluetooth, ZigBee, etc. In some exemplary embodiments of the present disclosure, the Controller 114 can comprise one or more Subsystems (or modules) for analyzing the feedback. Exemplary Controller Subsystems can include an artificial intelligence implemented using a neural network, a clinical decision Support System, and/or a machine vision System. In some exemplary cases, the machine vision System can be configured to perform image recognition based upon the feedback. Exemplary methods for image recognition can include edge detection, comer detection, and/or blob detection.

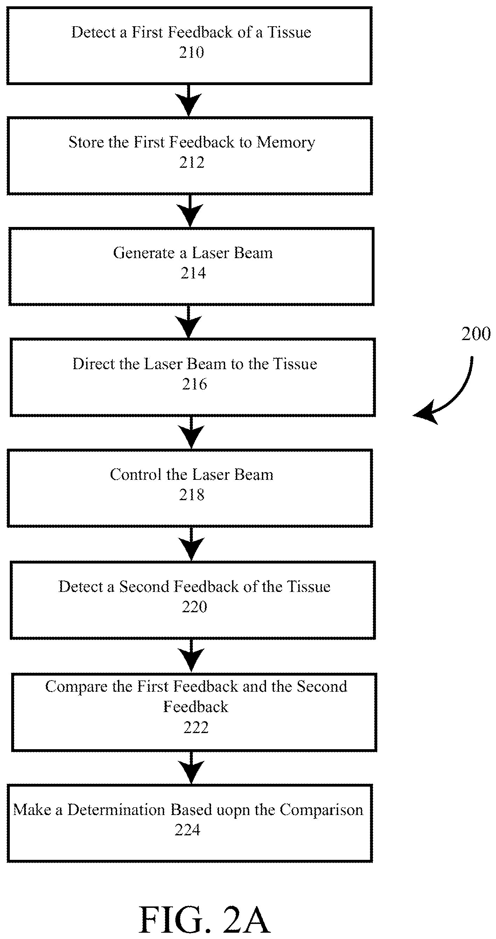

FIG. 2A illustrates a flow diagram of a method 200 for electromagnetic radiation (EMR)-based treatment, feedback detection, and analysis according to the exemplary embodiments of the present disclosure. As shown in Fig. 2A, a first feedback of a tissue can be detected in procedure 210. In some exemplary embodiments, a detector can be used to detect the first feedback. Exemplary detectors can include, but not limited to, e.g., a camera, an ultrasound transducer, a photoacoustic imaging System, an optical coherence tomography System, a photodiode, an optical coherence elastography System, a coherent anti-stokes Raman spectroscopy imaging System, a two-photon imaging System, second harmonic generation imaging System, a phase conjugate imaging System, a hyperspectral imaging System, a low-power carbon-dioxide laser imaging System, X-ray backscatter imaging System, a millimeter wave imaging System, a magnetic resonance imaging System, a high-frequency ultrasound imaging System, a photodiode, an ultrasound transducer array, a fluoroscope, a surface profilometer, an infrared imaging System, and/or a confocal microscope. Exemplary first feedback(s) can include one or more of digital images, digitized analog signals from analog sensors, subject feedback, etc. The exemplary first feedback can represent an exemplary aspect related to the subject, for example, a feedback that can includes a digital image can be used to represent targeted lesions within the tissue. Other exemplary aspects that can be represented by the first feedback(s) can include blood perfusion, tissue hydration, radiation absorption/scatter, tissue elasticity, and/or subject pain score.

The first feedback can then be stored to a computer/digital storage device (one or more memory device(s), etc.) in procedure 212. In some exemplary embodiments, the computer/digital storage device(s) can be located remotely from the exemplary System 100 and/or the treatment Subsystem 110, and can communicate with the computer/digital storage device(s) via one or more networks. Further, an electromagnetic radiation (EMR) beam can be generated in procedure 214. The EMR beam can then be directed to the tissue in procedure 216. The EMR beam in some exemplary embodiments can comprise a laser beam. The EMR beam can be directed to the tissue in order to perform a therapeutic, cosmetic, and/or aesthetic treatment of the tissue. One or more of characteristics of the EMR beam can be controlled in procedure 218 in response to the first feedback. The exemplary characteristics of the EMR beam that can be controlled can include a scan pattern, a pulse duration, a pulse energy, a repetition rate, and/or a wavelength.

According to some exemplary embodiments, a second feedback of the tissue can then be detected in procedure 220. The second feedback is some cases is then stored in and/or to the computer/digital storage device. The second feedback can then be compared to the first feedback in procedure 222. In some exemplary embodiments, the first and second feedback(s) can comprise images, and the comparison between the two images can be performed either manually or automatically. For example, in the exemplary cases where a pigmentary condition is being treated, the Controller can automatically compare pigmentation in the second feedback from the first feedback. This exemplary comparison can then be used to make a determination in procedure 224. For example, if the second feedback is found to contain less pigmentation than the first feedback, it can be determined that treatment for the pigmentary condition is progressing well. In some exemplary embodiments, subsequent feedbacks can be detected and stored to the computer/digital storage device to provide an electronic health record.

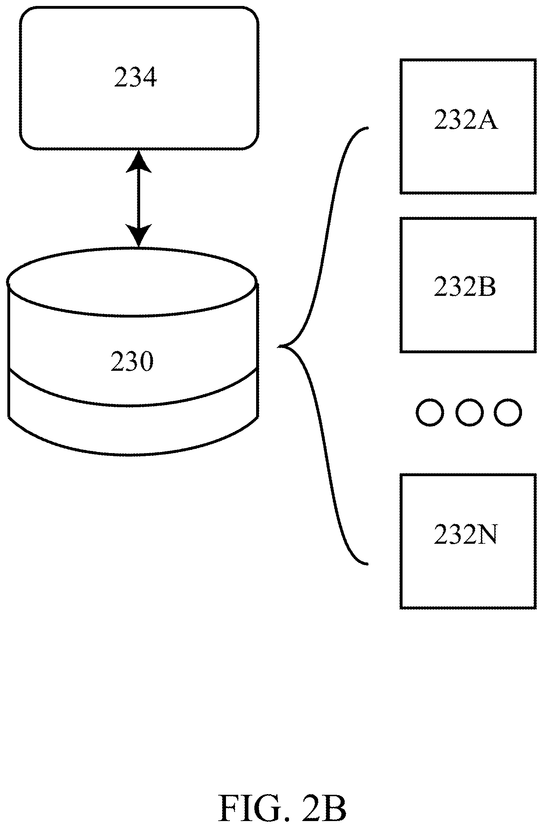

FIG. 2B shows a diagram of a data storage configuration 230 with containing multiple data entries 232 therein, according to certain exemplary embodiments of the present disclosure. The data entries 232 can each correspond to different feedbacks detected from the tissue. Typically, these exemplary feedback can be detected at different times, for example, before, during, and/or after treatments or between treatments. Time between the exemplary tissue feedback detection can range from seconds (e.g., 1 - 1000 seconds), hours (e.g., 1-24 hours), days (e.g., 1-7 days), weeks (e.g., 1-6 weeks), months (e.g., 1 - 12), or even years (e.g., 1-100 years). As shown in FIG. 2B, a Controller 234 can be provided in communication with the data storage configuration 230. The exemplary Controller 234 can have access to the data entries 232.

In some exemplary embodiments, the Controller 234 and the data storage 230 can be constituents of or facilitate a generation of an electronic health record (EHR) (i.e., electronic medical record [EMR]). In some exemplary embodiments, each individual EHR can correspond to an individual subject.

Depending on circumstance, exemplary feedbacks can be detected at different times relative to the exemplary treatment(s). For example, a pretreatment feedback can be detected prior to the exemplary treatment(s). Further, an intra-treatment feedback can be detected during treatment(s), and post-treatment(s). A pretreatment feedback, in some exemplary embodiments, can be used as a basis for EMR Parameter selection during treatment.

For example, an image of a tissue taken prior to treatment can be used to located lesions to be treated and estimate required EMR settings to achieve desired results at those locations. An image of a tissue taken during treatment can compare the location being treated with an actual lesion location and correct for errors during the treatment. Finally, an image of a tissue taken post-treatment can capture exemplary results of treatment. Exemplary EMR-based treatments often impart some damage or disruption to tissue through radiation, and titrating radiation is necessary for successful treatment in many cases. Documenting a tissue response after treatment can inform future treatments and documents treatment progress. Processing data derived from multiple feedbacks, in some exemplary embodiments, can assist in treatment. For example, in some exemplary embodiments, data from multiple feedbacks can be analyzed to determine one or more of the following: (i) if progress is being made, (ii) that safety is being ensured, (iii) that treatment parameters are being optimized, and (iv) that a time between treatments is being optimized. In some exemplary cases, only feedbacks from an individual undergoing the treatment can be used to inform treatment parameter selection. Alternatively or in addition, feedbacks from more than one subject may be used to inform treatment parameter selection. For example, in some exemplary embodiments, procedures (e.g., those incorporating machine learning, or artificial intelligence [Al]) use data derived from multiple feedbacks from many subjects in order to predict future optimal treatment Parameters or Support in clinical decision making.

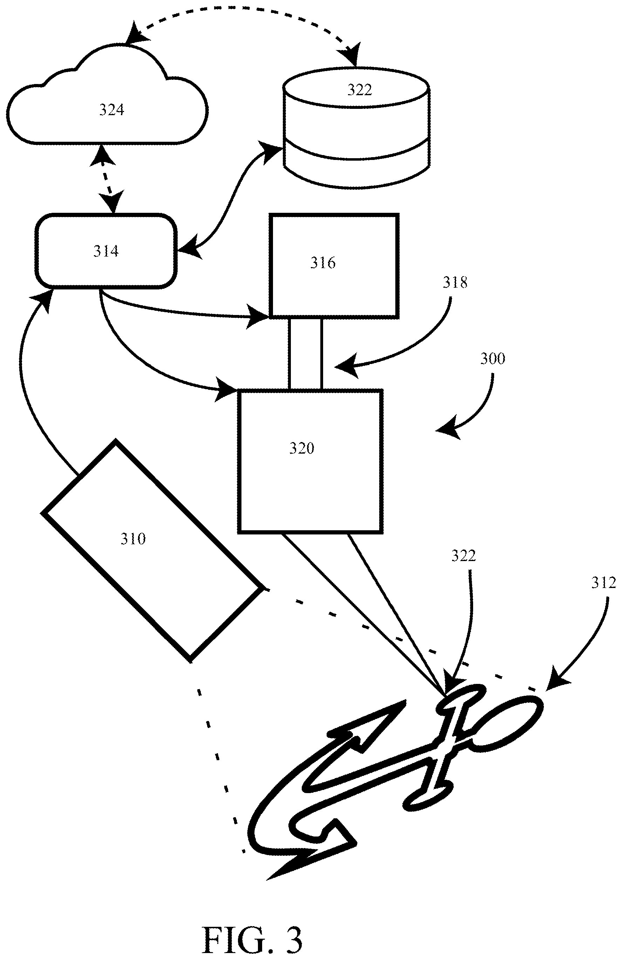

FIG. 3 shows a diagram of a feedback-controlled fractionated tattoo removal System 300 and exemplary interaction between exemplary components thereof, according to certain exemplary embodiments of the present disclosure. For example, the exemplary System 300 can include a sensor 310 which can be configured to sense a tattoo 312. Examples of sensors can include cameras (charge coupled device [CCD] and complementary metal-oxide semiconductor [CMOS]), photodiodes, and/or ultrasonic transducers. An exemplary camera can be a Pixel Link PL-D755 from Pixel Link of Ottawa, Ontario, Canada. The System 300 can include a Controllers 14 which can receive input from the sensor 310, and determine a location of the tattoo 312. In some exemplary embodiments, the sensor 310 can recognize and/or distinguish the tattoo 312 from the surrounding tissue. Other exemplary methods for detection can include known Computer vision methods and for brevity are not listed herein. The System can also include a laser source 316 which can generate a laser beam 318. Examples of a laser source 316 can include gas lasers (carbon dioxide, excimer, Helium Neon, etc.), solid-state lasers (diode-pumped solid state lasers [DPSS], optically pumped lasers), fiber optic lasers, and/or Q-switched lasers. According to some exemplary embodiments, the laser source can be a carbon dioxide (CO2) laser, for example a model PI 00 laser from Synrad of Mukilteo, Washington, U.S.A. For example, the PI 00 laser can produce a laser beam 318 having a wavelength of about 10.6pm with a peak power of about 400W nominal, a maximum average power of about 100W nominal, a maximum pulse duration of about 600pS, and a repetition rate from 0 to 100kHz.

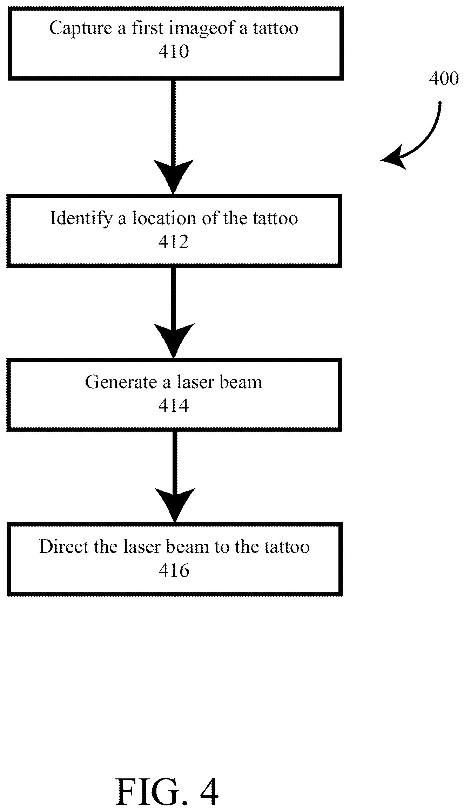

As shown in FIG. 3, the laser beam 318 can be directed to be incident on a laser beam scanning System 320. An example of the beam scanning System 320 can be the ProSeries 3-axis scan System from Cambridge Technology of Bedford, Massachusetts, U.S.A. In some exemplary embodiments, the laser beam scanning System 320 can also focus the beam to a focal region 322. A width of the focal region 322, in some exemplary cases, can be selected to be less than a predetermined size (e.g., less than 1mm, less than 500pm, less than 250pm, or less than 150pm) in order to minimize or otherwise reduce scarring. The beam scanning System 320 and the laser source 316 can both be controlled by the Controller 314. The exemplary Controller can be the ScanMaster Controller (SMC) from Cambridge Technology of Bedford, Massachusetts, U.S.A. In some exemplary embodiments, the Controller 314 can includes a laser and Scanner Controller (e.g., the SMC), as well as another processing device for performing taking input from the sensor 310. The Controller 314, in some exemplary cases, can store digital data representing Information from the sensor 310 or Information related to treatment to a data storage device 322. Examples of the digital data can include images of the tissue or tattoo, Coordinates treated by the laser, and location of reference marks (fiducials). In some exemplary cases, the Controller 314 can communicate with the data storage device 322 via one or more networks 324. Exemplary data storage device(s) 322 can include a hard disk drive and/or a network enabled data storage System. Exemplary networks can include local area networks (LAN), wide area networks (WAN), wireless networking technologies (Wi-Fi), internet, and/or cloud. In some exemplary cases, the exemplary System 300 can connect to the network(s) 324 with one or more network interface adapters (e.g., network interface card [NIC]). The Controller 314 can direct and activate the laser beam at predetermined locations over the tattoo 312 in order to perform the laser tattoo removal treatment. The exemplary treatment is further explained below in reference to FIG. 4.

FIG. 4 illustrates a flow diagram of a method 400 for a feedback-controlled ablative fractional laser tattoo removal, according to some exemplary embodiments of the present disclosure. For example, the System 300 can captures a first image of the tattoo in procedure 410. The first image of the tattoo - in some exemplary cases - can include at least part of the tattoo (not necessarily all of the tattoo). In some exemplary cases, one or more additional images of the tattoo can be captured.

Further, a location of the tattoo can be identified using the first image in procedure 412. In some exemplary embodiments, the tattoo can be recognized in the first image using machine vision technique(s). For example, in some exemplary cases, edge detection and/or feature detection can be used to recognize the tattoo within the first image. Exemplary edge detection methods can include Canny edge detection, Sobel edge detection, Shi and Tomasi comer detection, and features from accelerated segment test (FAST) comer and/or blob detection. In other exemplary embodiments, machine learning techniques can be employed to recognize the tattoo within the first image. Exemplary machine learning methods can include neural networks and TensorFlow from Google of Mountain View, California, U.S.A. For example, when the tattoo is recognized within the first image, its location relative the System 300 can be determined. In some exemplary embodiments, a homography transform can be used to determine the location of the tattoo relative the camera. In some other exemplary embodiments, a second camera to capture a second image and stereoscopic calculations can be used to determine a relative position of the tattoo. Once a location of the tattoo relative to the System 300 is known, a laser beam can be directed to precise locations of the tattoo.

In such exemplary Situation, a laser beam can be generated by a laser source in procedure 414. Examples of laser sources are described in detail herein. In concert with the generation of the laser beam of procedure 414, the laser beam can be directed to the tattoo 416. For example, the laser beam can be directed to a multitude of locations at the surface of the tattoo. In some exemplary embodiments, the multitude of locations can be separated from one another by a distance (i.e., pitch) in order that only a fraction (e.g., 10%, 30%, 40% or 50%) of the tattoo is ablated during laser treatment.

Unlike other methods of laser tattoo removal, this exemplary method according to certain exemplary embodiments of the present disclosure does not rely on selective photothermolysis. For example, the exemplary method does not need to use a laser beam having a wavelength that is absorbed (selectively) by tattoo pigment and not by surrounding tissue. Instead, the exemplary method of tattoo removal according to certain exemplary embodiments of the present disclosure can utilize a laser having a wavelength that is absorbed by tissue directly (e.g., 200 - 500nm, 1300 - 3500nm, and/or 9-1 Ipm) and ablate tissue containing pigment. The Controller 314 can direct the laser to the tissue in a fractionated (i.e., only irradiating a fraction of the region) manner in order to remove the tattoo at the irradiated locations. The exemplary ablative fractional laser treatment, which can be performed both with and without conventional Q-switched laser tattoo removal, has been demonstrated in the literature without feedback-control. In a publication by Ibrahimi et al., it was indicated that ablative fractional resurfacing can be safe and effective in removal of allergic tattoos, when used with a Q-switched laser in Treatment of Tattoo Allergy with Ablative Fractional Resurfacing: a Novel Paradigm for Tattoo Removal - published in the Journal of the American Academy of Dermatology in June 2011. In another publication (by Seitz et al., "Fractional CO2Taser is as Effective as Q-Switched Ruby Laser for the Initial Treatment of a Traumatic Tattoo". Journal of Cosmetic Laser Therapy, December 2014), it was indicated that "in the initial stage of removing the traumatic tattoo, the ablative fractional laser treatment appeared to be as effective as the Standard ruby laser therapy," in. However, in the Seitz et al. publication, it was commented that after the initial treatment or two ablative fractional treatment failed to maintain efficacy when compared to the Q-switched tattoo laser removal laser. This is because, typically laser fractional treatment delivers an array of focused laser spots to a tissue without determining the exact location for each individual spot. Typically, a pitch between adjacent spots or a fill factor (ratio of ablative area to total area) is controlled, but not the precise location of each individual laser spot. As a result, multiple fractional treatments of the tattoo treat the same locations repeatedly while failing to treat only the areas of the tattoo that remain. The failure demonstrated in the Steiz et al. publication can be overcome by the exemplary methods and System according to the exemplary embodiments of the present disclosure. In comparison, the exemplary Systems and methods according to the exemplary embodiment of the present disclosure can recognize and map the tattoo to target only locations where the tattoo persists in each treatment.

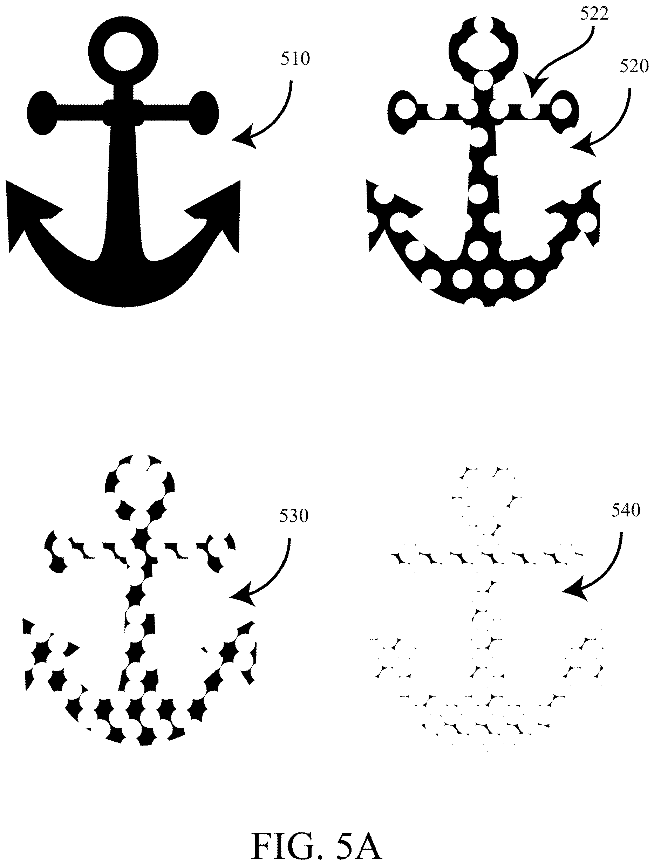

FIG. 5A illustrates a tattoo as it undergoes three different treatments, according to some exemplary embodiments of the present disclosure. As shown in FIG 5A, an untreated nautical-themed tattoo 510 is provided in an upper comer, and a post-first-treatment image 520 of the tattoo is shown opposite to the untreated tattoo 510. The post-first-treatment image 520 illustrates the tattoo after it has undergone a first treatment 400. A multitude of ablated regions 522 is provided in which a fraction of the tattoo has been removed during the first treatment 400. During a second treatment, a second multitude of ablated regions can be used to remove another fraction of the tattoo. However, as the exemplary System 300 is feedback-controlled, the laser can be precisely directed to locations that have not been previously ablated. A post-second-treatment image 530 of the tattoo provides a second multitude of ablated regions, not overlapping, but adjacent to the original set of ablated regions. Further, a post-third-treatment image 540 of the tattoo provides a third multitude of ablated regions adjacent to the first and second set of ablated regions. The post-third-treatment image of the tattoo 540 illustrates that the tattoo is almost completely removed in three treatments. In comparison, most Professional tattoos are not removed even after 5 treatments using the prior laser tattoo removal treatment Systems and methods.

An exemplary table below compares a feedback-controlled fractional ablative tattoo removal with a non-feedback-controlled fractional ablative tattoo removal. The table assumes both fractional treatments use a one-third (e.g., 33%) fill factor, so that approximately one third of the tattooed area is ablated in each treatment. However, without a precise placement of the laser beam ensuring that only previously untreated locations are targeted by the laser, conventional fractional approaches can be seen as requiring many more treatments and likely become less effective with each additional subsequent treatment.

**Table 1 Exemplary Feedback -Contralled Treatment vs. Conventional Fractional Laser Treatment for Tattoo Removal**

| **Treatment No.** | **Feedback Controlled (No Overlap)** | | **Conventional Fractional (Statistical Overlap)** | |
|---|---|---|---|---|
| | **Tattoo Remaining Fraction** | **Tattoo Remaining Percentage** | **Tattoo Remaining Fraction** | **Tattoo Remaining Percentage** |
| **1** | 2/3 | 67% | 2/3 | 67% |
| **2** | 1/3 | 33% | 4/9 | 44% |
| **3** | 0 | *0%* | 8/27 | 30% |
| **4** | | | 16/81 | 20% |
| **5** | | | 32/243 | 13% |
| **6** | | | 64/729 | 9% |
| **7** | | | 128/2187 | 6% |
| **8** | | | 256/6561 | 4% |
| **9** | | | 512/19683 | 3% |
| **10** | | | 1032/59049 | 2% |

Another exemplary benefit to a feedback-controlled fractional laser tattoo removal according to the exemplary embodiments of the present disclosure can be that pigment leaves the skin directly during treatment. For example, during the ablation, the pigment (e.g., tattoo ink) is expelled from the tissue (e.g., as a gas or particles within a gas). After the ablation, pigment may further be expelled through the channels left behind (i.e., exudate) in the tissue and through sluffing off of heat affected (e.g., coagulated) tissue that is not ablated. A direct removal of tattoo inks can be beneficial because conventional procedure(s) of tattoo removal rely on biological functions to remove the tattoo ink through internal ways, and the composition of tattoo inks are largely unregulated. These biological functions that aid in the tattoo removal are not widely understood, although it is know that the tattoo ink is removed from the dermis of the tissue and travels to other parts of the body (for example, in lymph). As the composition of the inks is largely unknown and potentially toxic, it is preferred that removal of the tattoo from the dermis does not simply relocate the inks to other parts of the body, but removes the tattoo from the body directly.

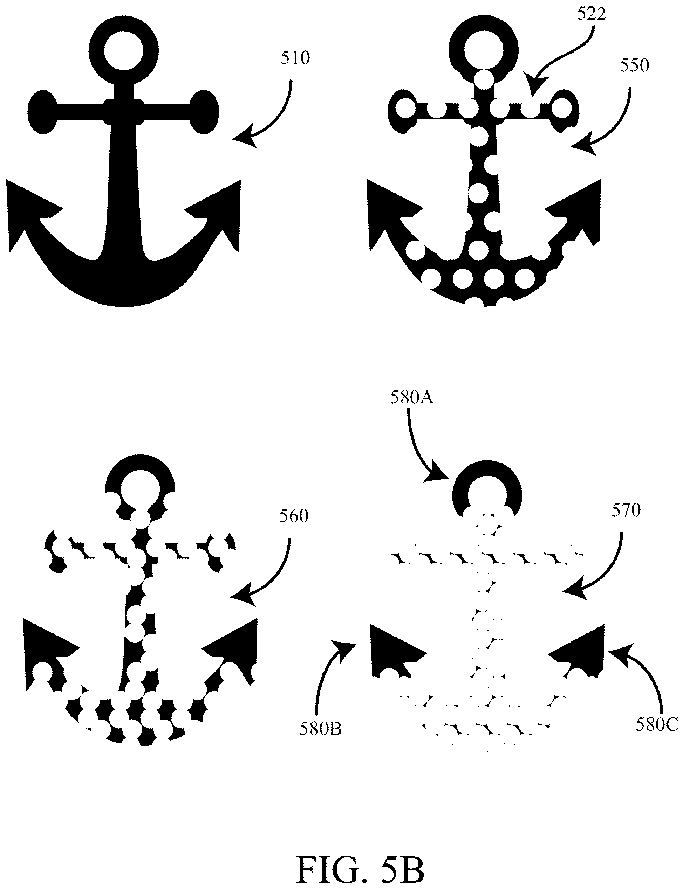

According to some exemplary embodiments of the present disclosure, registration of the tattoo is required in order to ensure that only untreated areas of the tattoo ablated. In one exemplary case, as shown in FIG. 5B, some small fiducial markings 580A-580C are left purposely unremoved during the tattoo removal process. These fiducial markings 580A-580C can be the remnants of the tattoo and used to locate (i.e., register) the tattoo before each treatment. In particular, FIG. 5B illustrates, e.g., the same fractionated tattoo removal process shown in FIG. 5A, except with fiducial remnants 580A-580C. An untreated nautical-themed tattoo 510 is shown in an upper comer of FIG. 5B. A post-first-treatment image 550 of the tattoo is provided opposite the untreated tattoo 510. The post-first-treatment image 550 illustrates the tattoo after it has undergone the first treatment 400. A multitude of ablated regions 522 have removed a fraction of the tattoo during the first treatment. During a second treatment, a second multitude of ablated regions are used to remove another fraction of the tattoo. A post-second-treatment image 560 of the tattoo shows a second multitude of ablated regions, not overlapping, but adjacent to the original set of ablated regions. Further, a post-third-treatment image 570 of the tattoo shows a third multitude of ablated regions adjacent to the first and second set of ablated regions. The post-third-treatment image of the tattoo 570 shows that the tattoo is almost completely removed, except for the three fiducials/markings 580A-580C, in three treatments.

Other exemplary embodiments provide fractional feedback-controlled treatments for different conditions. For example, in one exemplary embodiment, a fractional treatment can be performed on a subject for skin rejuvenation and the location of the fractional treatment regions on the subject's skin is stored in the digital storage device. During subsequent treatments, the locations of previous fractional treatment regions can be used to control the exemplary EMR-based treatment System 110, and can direct fractionated treatment to new regions that do not overlap with the previous treatment regions.

Fractional tattoo removal is described in detail herein, and additional therapeutic, aesthetic, and/or cosmetic treatments can be performed with the exemplary embodiments of the present disclosure. For example, according to some exemplary embodiments, the exemplary methods, Systems and computer-accessible medium can be used to perform laser hair removal.

Typically, laser hair removal treatments generally utilize wavelengths that are in the visible or near infrared spectrum (NIR). These wavelengths are absorbed by melanin containing structures as are found in dark hair and skin. Hair removal therefore works well for subjects who have dark (e.g., brown) hair and fair skin (e.g., Fitzgerald skin type of two or fewer). Laser hair removal is therefore seldom recommended for those with dark skin types (e.g., Fitzgerald skin type of three or more). Additionally, subjects with blond, white, and/or light red hair may also not be good candidates for a conventional laser hair removal. The present disclosure described various exemplary embodiments of a method for laser hair removal which can be utilized for such previously unserved subjects.

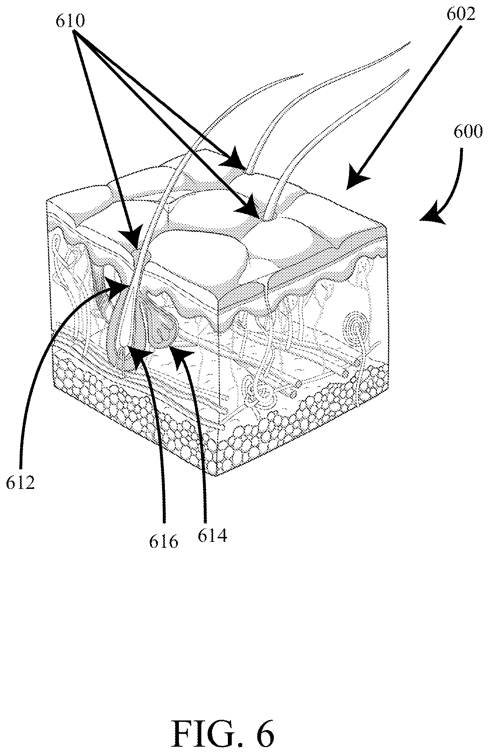

For example, FIG. 6 shows a sectional view 600 of skin tissue 602 which can be effectuated by the exemplary methods and Systems according to the exemplary embodiments of the present disclosure. According to some exemplary embodiments, an image of the tissue is first taken and used to locate hair shafts 610. The image can then be analyzed in order to locate hair shafts within the image. In some exemplary embodiments, machine learning algorithms/procedures and/or machine vision techniques can be utilized in order to identify and locate the hair shafts. The location of the hair shafts in the image can then be used to determine a location of the hair shafts relative the System 100. Exemplary methods for such exemplary Implementation are described herein, and can include homography transform, and stereoscopic calculations.

Once the locations of the hair shafts 610 are determined relative the exemplary System 100, such exemplary System 300 can direct a laser beam generally toward hair follicles 612 associated with each hair shaft 610 to damage and/or disrupt the growth of hair. Hair follicles are generally provided at an angle relative to the skin's surface, and an angle of a protruding hair does not necessarily predict the angle of the hair follicle. However, the angle of the hair follicle likely has a distribution that can be determined or estimated. In some exemplary embodiments, a high-magnification camera (e.g., 100x- 200x) can be used to automatically locate where each individual hair exits the skin. Treatment locations that are statistically likely to overlap with each hair follicle can then be determined based upon the location where each individual hair exits the skin and a distribution of the angle(s) of the hair follicle(s). The treatment locations can then be selectively irradiated by the exemplary treatment System 110. The laser beam delivered to each hair follicle does not need to be selectively absorbed by the hair follicle 612. This is unlike conventional forms of laser hair removal treatments currently on the market that require a laser be used that has a wavelength which is absorbed by the hair follicle 612 and not the surrounding skin 602. Instead, according to the exemplary embodiments of the present disclosure, the laser beam directed to each individual hair follicle 612 can be any type of laser with sufficient energy to damage and/or disrupt the function of the hair follicle 612. Spatially selective laser delivery based on feedback can be used to treat other conditions by delivering energy to areas near hair follicles.

According to some exemplary embodiments of the present disclosure, feedback-controlled hair removal can be performed in fewer treatments than conventional laser hair removal, which typically takes three sessions. Current laser hair removal treatments most appropriately target hairs in an anagen phase. Hairs generally experience three growth phases including anagen, catagen, and telogen. For this reason, only about one third of all hairs are in an anagen growth phase at any given time. For this reason, conventional laser hair removal typically generally requires about three treatments. In contrast, beneficially, the exemplary feedback-controlled hair removal according to various exemplary embodiments of the present disclosure can target the hair bulb 616, directly, not the hair shaft in the anagen phase (like conventional laser hair removal). Therefore, in some exemplary embodiments, feedback-controlled laser hair removal can be effective in only one treatment session.

According to another exemplary embodiment of the present disclosure, it is possible to utilize the exemplary System and method to treat acne vulgaris (acne). Acne is caused by a blockage of the hair follicle 612. This blockage is caused in part by sebum from a sebaceous gland 614. It has been described in U.S. Patent Application Serial No. 10/612,599, that disruption of the sebaceous gland 614 through laser radiation can effectively treat (and perhaps eure) acne. By disrupting the sebaceous gland 614, the flow of sebum from the hair follicle 612 can be slowed or even stopped. Without sebum, blockages of the hair follicles 614 (and acne blemishes) likely do not occur. Referring again to FIG. 6, sebaceous glands 614 are located proximal to the hair shafts 610. In some exemplary embodiments, sebaceous glands can be located and targeted by the laser System in order to arrest the flow of sebum and treat acne. In addition to disrupting sebaceous glands, the above describe exemplary techniques according to some exemplary embodiments of the present disclosure can be used to target and disrupt other glands including eccrine glans (for example to treat hyperhidrosis) and apocrine glands (for example to treat bromhidrosis). In some exemplary embodiments, lesions (e.g., glands, hair follicles, etc.) can be treated (e.g., damaged or disrupted) through ablation (for example with a 10,600nm CO2 laser source). In other exemplary embodiments, the treatment achieved through coagulation (for example, with a 1550nm fiber laser source). In still other exemplary embodiments, treatment can be purely photo-induced (non-thermal) (e.g., with a 248nm excimer laser). Other exemplary dermatological treatments can be utilized that can utilize the exemplary feedback-controlled treatment to deliver the EMR in a predetermined pattern to the skin tissue.

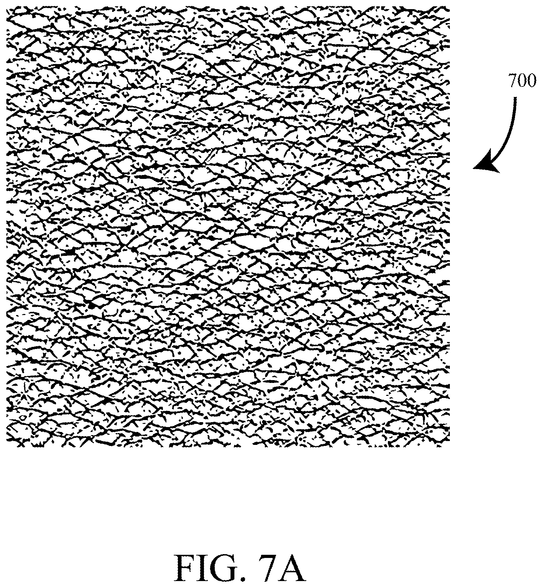

According to some exemplary embodiments of the present disclosure, it is possible to resurface scar tissue. Focused laser beams having a short wavelength (e.g., less than 500nm) can be used to make lasting (e.g., permanent) modifications to a surface of skin tissue. Scar tissue on the skin generally does not contain hair follicles, pores, or normal skin texture. As a result, skin scar surfaces likely stand out in contrast against normal unscarred skin. According to some exemplary embodiments, the exemplary System 100 can be used to make permanent textural modifications to a surface of skin scar tissue to make it appear more like unscarred skin. FIG. 7A illustrates a vector-based graphic 700 of skin tissue. According to some exemplary embodiments, the graphic 700 can be used as a scan pattern to mark the surface of skin scar tissue. As a result, a texture based upon the graphic 700 can be imparted to the scar tissue, causing the scar tissue to appear more like the healthy skin that surrounds it. Other types of scars can also be treated using the exemplary feedback controlled laser-based treatments according to the exemplary embodiments of the present disclosure.

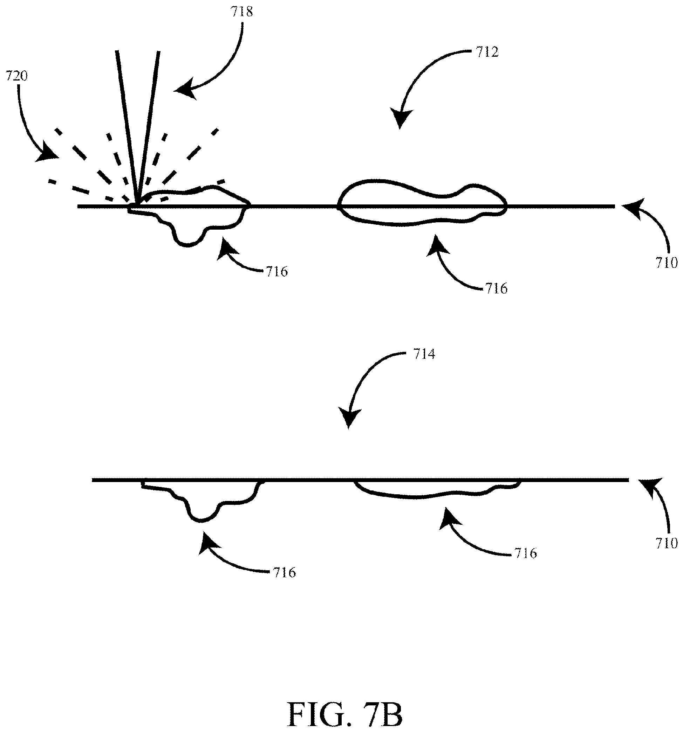

FIG. 7B shows an Illustration of a tissue 710 (e.g., pre-treated tissue 712 and post-treated tissue 714) which was subjected to an exemplary feedback controlled scar resurfacing treatment according to certain exemplary embodiments of the present disclosure. Hypertrophie scars 716 (e.g., raised acne scars) are typically raised above a surface of the tissue 710. According to some exemplary embodiments, a laser beam 718 can be directed selectively toward the hypertrophic scars 716 to cause an ablation 720 and remodel the scars 716. In some exemplary embodiments, a scan path of the laser beam 718 can be controlled to direct the laser beam 718 only toward the scars 716. A sensor (e.g., profilometer) can detect a feedback from the tissue 710 and determine the scan path based upon the feedback. Direct ablative remodeling can be possible with smaller hypertrophic (e.g., acne) scars, however thick scars can require a different approach to remodeling.

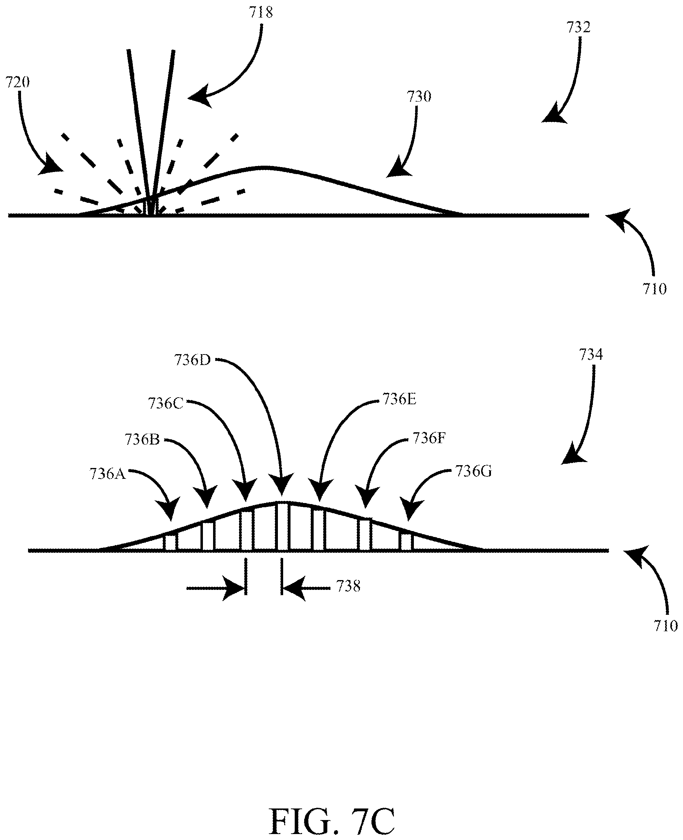

FIG. 7C shows an Illustration of a skin 710 with a thick scar 730 (e.g., pre-treated tissue 732 and post-treated tissue 734) subjected to a fractionated feedback-controlled laser treatment according to certain exemplary embodiments of the present disclosure. The exemplary fractionated feedback-controlled laser treatment can produce an array of channels 736A-736G within the thick scar 730. In some exemplary embodiments, a scan path of the laser beam 718 can be controlled to direct the laser beam 718 only toward the thick scar tissue 730. A sensor (e.g., profilometer) can detect a feedback from the tissue 710 and determine the scan path based upon the feedback. Additionally, in some exemplary embodiments, another laser parameter can be controlled based upon a feedback related to a thickness of the thick scar 730. For example, a pulse energy in some cases is varied based upon the thickness of the thick scar tissue 730. This can facilitate the treatment to produce longer channels 736C - 736E through thick sections of the thick scar and shorter channels 736A, 736B, 736F, and 736G, where the scar 730 is less thick. In another exemplary embodiment, a density of channels or a pitch 738 between channels can be varied based upon the thickness of the scar 730. For example, in some exemplary versions, the pitch 738 between channels can be less in thick sections of the scar 730 and greater where the scar 730 is thinner. In addition to thick scars, the exemplary treatment as described herein with reference to FIG. 7C can be used in connective tissue remodeling, for example, to treat cellulite (e.g., striae).

In some exemplary embodiments, the above-described exemplary feedback-controlled resurfacing techniques can be used to treat any of a myriad of conditions including bums, keloids, hypertrophic scars (e.g., surgical), atrophic scars (e.g., pockmark acne scars), chickenpox or measles scars, smallpox vaccine scars, wrinkles, and striae (e.g., stretch marks).

### Further Exemplary Embodiment(s)

An exemplary System according to further exemplary embodiments of the present disclosure is described herein. For example, the exemplary System can includes a 3 axis CO2 laser marking System (Model No. Z9600 from Keyence Corporation of America of Itasca, Illinois, U.S.A.) for delivering electromagnetic radiation (EMR). The exemplary System can also include a USB camera (Model No. PL-D755 from Pixel Link of Ottawa, Ontario, Canada), which is used to provide feedback. The exemplary laser marking system/apparatus and the camera can both be connected to a Computer running Matlab (from MathWorks of Natick, Massachusetts, U.S.A.). In one exemplary Implementation, first Matlab script can be implemented to utilize images captured by the camera to register an object and recognize targets within the images. A second Matlab script can be used to perform a homography transform of the images from an image space to a laser marking System space. Further, Irradiation target Coordinates can be transmitted to the laser marking System and the laser selectively irradiates the targets.

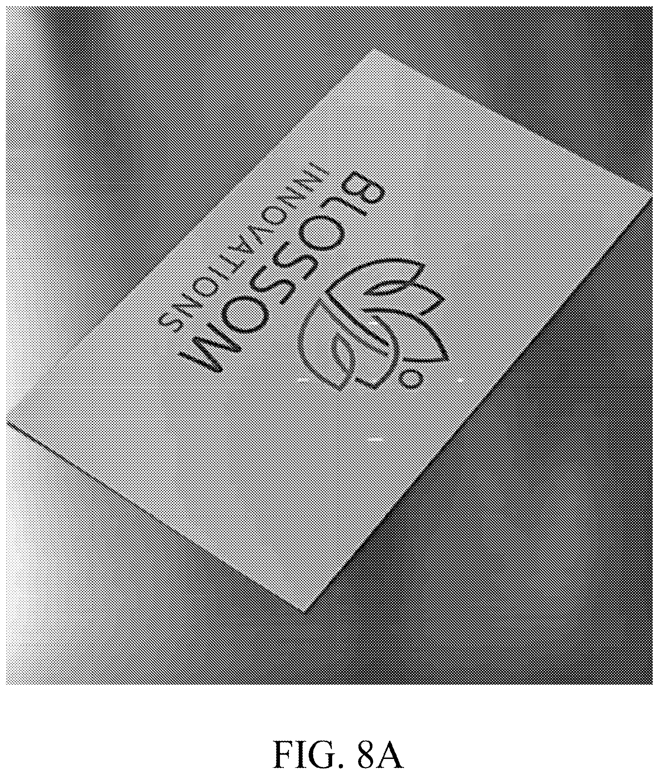

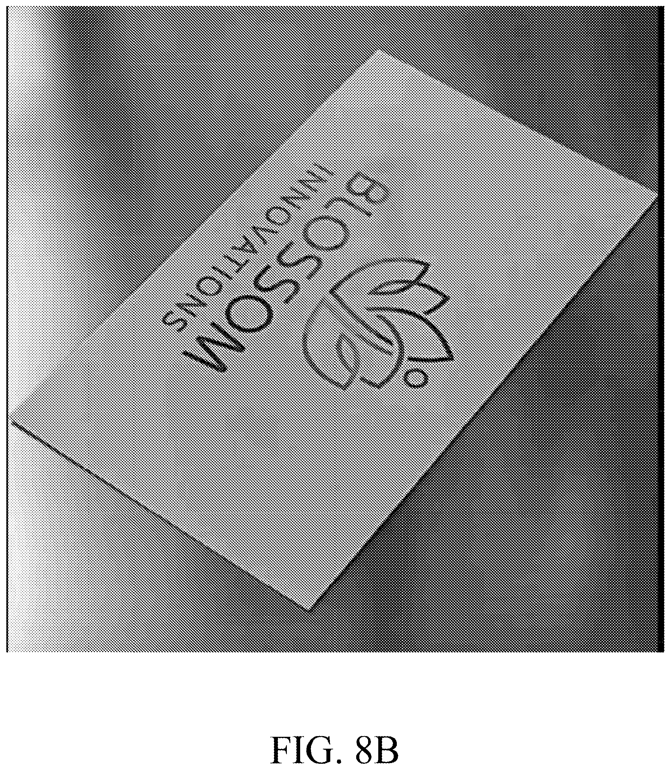

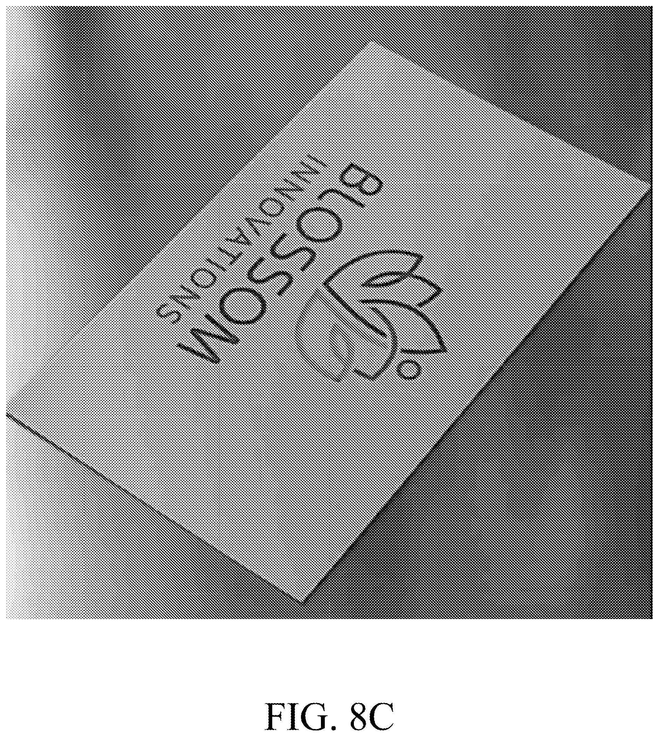

FIGS. 8A-8C illustrate exemplary images showing the exemplary System according to various exemplary embodiments of the present disclosure being utilized to selectively target regions of a business card for laser Irradiation according to certain exemplary embodiments. For example, FIG. 8A shows an image of a number of visible diode laser dots from the laser marking System displayed on a surface of the business card. During this exemplary Step, the exemplary System can register the location of the business card. FIG. 8B shows 'green' regions on the Business card being targeted by the 3D marking System. Prior to this procedure, the prototype System recognized 'green' or bright regions of the business card, and can select them for Irradiation. Further, FIG. 8C illustrates an image of the business card after all of the 'green' or bright regions on the business card have been irradiated.

Additionally, the exemplary feedback-controlled laser treatment in some exemplary embodiments is used to treat vascular lesions (e.g., leg veins and telangiectasia). For example, in some exemplary embodiments, the exemplary feedback System 112 can register one or more locations of certain vascular lesions (e.g., leg veins) and the exemplary treatment System 110 can direct an EMR-based treatment to the vascular regions.

Additional exemplary embodiments can include alternative feedback technologies used in conjunction with various EMR-based treatment. These alternative imaging technologies can include, e.g., microscopic imaging, wide field of view imaging, reflectance confocal imaging, optical coherence tomography imaging, optical coherence elastography imaging, coherent anti-stokes Raman spectroscopy imaging, two-photon imaging, second harmonic generation imaging, phase conjugate imaging, photoacoustic imaging, infrared spectral imaging, and/or hyperspectral imaging.

In some additional exemplary embodiments, the exemplary feedback-controlled treatments can employ selective photothermolysis. For example, the exemplary treatment System same as or similar to those currently used (for example for tattoo and hair removal), in some exemplary embodiments, can be configured to operate in concert with an exemplary feedback System of the exemplary embodiments of the present disclosure to automatically perform a treatment. For example, the exemplary System 100 can locate the lesion, optionally determine one or more treatment parameters, and direct the treatment toward the lesions. In some further exemplary embodiments, the exemplary System 100 can then image the tissue response and adjust parameters accordingly. As an example, laser hair removal today requires a practitioner to manually direct a laser beam to every hair follicle. According to some exemplary embodiments, the laser can be automatically directed to each follicle instead of manually. Treatments that are suitable for automation through feedback-controlled laser treatment can include those treatments that are effective using current technologies and are currently performed manually, including hair removal, treatment of vascular lesions (e.g., port-wine stains and rosacea), and pigmented lesions. Exemplary laser sources for the automated feed-back controlled photothermolysis treatments can include those that target selected chromophores, such as 755nm alexandrite, 1064nm Nd:YAG, 532nm second harmonic of Nd:YAG, 595nm pulsed dye laser, and 808nm diode laser.

Although treatments for some exemplary conditions have been described above, it should be understood that the exemplary Systems, methods and devices according to exemplary embodiments of the present disclosure can be suitable for treating any number of medical, aesthetic, and cosmetic conditions. Exemplary additional conditions can include gastrointestinal (Gl) tract abnormalities and vaginal rejuvenation.

One skilled in the art will appreciate further features and advantages of the present disclosure based on the above-described embodiments. Accordingly, the invention is not to be limited by what has been particularly shown and described, except as indicated by the appended Claims.

The subject matter described herein can be implemented in digital electronic circuitry, or in Computer Software, firmware, or hardware, including the structural means disclosed in this specification and structural equivalents thereof, or in combinations of them. The subject matter described herein can be implemented as one or more Computer program products, such as one or more Computer programs tangibly embodied in an Information carrier (e.g., in a machine readable storage device), or embodied in a propagated signal, for execution by, or to control the Operation of, data processing apparatus (e.g., a programmable processor, a Computer, or multiple Computers). A Computer program (e.g., also known as a program, Software, Software application, or code) can be written in any form of programming language, including compiled or interpreted languages, and it can be deployed in any form, including as a stand-alone program or as a module, component, subroutine, or other unit suitable for use in a computing environment. A Computer program does not necessarily correspond to a file. A Computer program can be stored or recorded in a portion of a file that holds other programs or data, in a single file dedicated to the program in question, or in multiple coordinated files (e.g., files that store one or more modules, sub programs, or portions of code). A Computer program can be deployed to be executed on one Computer or on multiple Computers at one site or distributed across multiple sites and interconnected by a communication network.

The exemplary processes, method, procedure and logic flows described in this specification, including the method steps of the subject matter described herein, can be performed by one or more programmable processors executing one or more Computer programs to perform functions of the subject matter described herein by operating on input data and generating Output. The processes and logic flows can also be performed by, and exemplary apparatus of the subject matter described herein can be implemented as, special purpose logic circuitry, e.g., an FPGA (field programmable gate array) or an ASIC (application specific integrated circuit).

Processors suitable for the execution of a Computer program include, by way of example, both general and special purpose microprocessors, and any one or more processor of any kind of digital Computer. Generally, a processor will receive instructions and data firoma read only memory or a random access memory or both. The essential elements of a Computer are a processor for executing instructions and one or more memory devices for storing instructions and data. Generally, a Computer will also include, or be operatively coupled to receive data from or transfer data to, or both, one or more mass storage devices for storing data, e.g., magnetic, magneto optical disks, or optical disks. Information carriers suitable for embodying Computer program instructions and data include all forms of non-volatile memory, including by way of example semiconductor memory devices, (e.g., EPROM, EEPROM, and flash memory devices); magnetic disks, (e.g., internal hard disks or removable disks); magneto optical disks; and optical disks (e.g., CD and DVD disks). The processor and the memory can be supplemented by, or incorporated in, special purpose logic circuitry.

To provide for interaction with a user, the subject matter described herein can be implemented on a Computer having a display device, e.g., a CRT (cathode ray tube) or LCD (liquid crystal display) monitor, for displaying Information to the user and a keyboard and a pointing device, (e.g., a mouse or a trackball), by which the user can provide input to the Computer. Other kinds of devices can be used to provide for interaction with a user as well. For example, feedback provided to the user can be any form of sensory feedback, (e.g., visual feedback, auditory feedback, or tactile feedback), and input from the user can be received in any form, including acoustic, speech, or tactile input.

The exemplary techniques described herein can be implemented using one or more modules. As used herein, the term "module" refers to Computing Software, firmware, hardware, and/or various combinations thereof. At a minimum, however, modules are not to be interpreted as Software that is not implemented on hardware, firmware, or recorded on a non-transitory processor readable recordable storage medium (i.e., modules are not Software per se). Indeed "module" is to be interpreted to always include at least some physical, non-transitory hardware such as a part of a processor or Computer. Two different modules can share the same physical hardware (e.g., two different modules can use the same processor and network interface). The modules described herein can be combined, integrated, separated, and/or duplicated to Support various applications. Also, a function described herein as being performed at a particular module can be performed at one or more other modules and/or by one or more other devices instead of or in addition to the function performed at the particular module. Further, the modules can be implemented across multiple devices and/or other components local or remote to one another. Additionally, the modules can be moved from one device and added to another device, and/or can be included in both devices.

The subject matter described herein can be implemented in a Computing System that includes a back end component (e.g., a data Server), a middleware component (e.g., an application Server), or a front end component (e.g., a client Computer having a graphical user interface or a web browser through which a user can interact with an Implementation of the subject matter described herein), or any combination of such back end, middleware, and front end components. The components of the System can be interconnected by any form or medium of digital data communication, e.g., a communication network. Examples of communication networks include a local area network ("LAN") and a wide area network ("WAN"), e.g., the Internet.

Approximating language, as used herein throughout the specification and paragraphs, may be applied to modify any quantitative representation that could permissibly vary without resulting in a change in the basic function to which it is related. "Approximately," "substantially," or "about" can include numbers that fall within a range of 1%, or in certain exemplary embodiments within a range of 5% of a number, or in certain exemplary embodiments within a range of 10% of a number in either direction (greater than or less than the number) unless otherwise stated or otherwise evident from the context (except where such number would impermissibly exceed 100% of a possible value). Accordingly, a value modified by a term or terms, such as "about," "approximately," or "substantially," are not to be limited to the precise value specified. In at least some instances, the approximating language may correspond to the precision of an Instrument for measuring the value. Here and throughout the specification and paragraphs, range limitations may be combined and/or interchanged, such ranges are identified and include all the sub-ranges contained therein unless context or language indicates otherwise.

The articles "a" and "an" as used herein in the specification and in the paragraphs, unless clearly indicated to the contrary, should be understood to include the plural referents. Paragraphs or descriptions that include "or" between one or more members of a group are considered satisfied if one, more than one, or all of the group members are present in, employed in, or otherwise relevant to a given product or process unless indicated to the contrary or otherwise evident from the context. The disclosure includes embodiments in which exactly one member of the group is present in, employed in, or otherwise relevant to a given product or process. The disclosure also includes embodiments in which more than one, or all of the group members are present in, employed in, or otherwise relevant to a given product or process. Furthermore, it is to be understood that the disclosed embodiments provide all variations, combinations, and permutations in which one or more limitations, elements, clauses, descriptive terms, etc., from one or more of the listed paragraphs is introduced into another claim dependent on the same base claim (or, as relevant, any other Claim) unless otherwise indicated or unless it would be evident to one of ordinary skill in the art that a contradiction or inconsistency would arise. It is contemplated that all embodiments described herein are applicable to all different aspects of the disclosed embodiments where appropriate. It is also contemplated that any of the embodiments or aspects can be freely combined with one or more other such embodiments or aspects whenever appropriate. Where elements are presented as lists, e.g., in Markush group or similar format, it is to be understood that each subgroup of the elements is also disclosed, and any element(s) can be removed from the group. It should be understood that, in general, where the disclosed embodiments, or aspects of the disclosed embodiments, is/are referred to as comprising particular elements, features, etc., certain embodiments of the disclosure or aspects of the disclosure consist, or consist essentially of, such elements, features, etc. For purposes of simplicity those embodiments have not in every case been specifically set forth in so many words herein. It should also be understood that any embodiment or aspect of the disclosure can be explicitly excluded from the paragraphs, regardless of whether the specific exclusion is recited in the specification. For example, any one or more active agents, additives, ingredients, optional agents, types of organism, disorders, subjects, or combinations thereof, can be excluded.

Where ranges are given herein, embodiments of the disclosure include embodiments in which the endpoints are included, embodiments in which both endpoints are excluded, and embodiments in which one endpoint is included and the other is excluded. It should be assumed that both endpoints are included unless indicated otherwise. Furthermore, it is to be understood that unless otherwise indicated or otherwise evident from the context and understanding of one of ordinary skill in the art, values that are expressed as ranges can assume any specific value or subrange within the stated ranges in different embodiments of the disclosure, to the tenth of the unit of the lower limit of the range, unless the context clearly dictates otherwise. It is also understood that where a series of numerical values is stated herein, the disclosure includes embodiments that relate analogously to any intervening value or range defined by any two values in the series, and that the lowest value may be taken as a minimum and the greatest value may be taken as a maximum. Numerical values, as used herein, include values expressed as percentages.

It should be understood that, unless clearly indicated to the contrary, in any methods claimed herein that include more than one act, the order of the acts of the method is not necessarily limited to the order in which the acts of the method are recited, but the disclosure includes embodiments in which the order is so limited. It should also be understood that unless otherwise indicated or evident from the context, any product or composition described herein may be considered "isolated".

As used herein the term "comprising" or "comprises" is used in reference to compositions, methods, and respective component(s) thereof, that are essential to the disclosed embodiments, yet open to the inclusion of unspecified elements, whether essential or not.

As used herein the term "consisting essentially of refers to those elements required for a given embodiment. The term permits the presence of additional elements that do not materially affect the basic and novel or functional characteristic(s) of that embodiment of the disclosure.

The term "consisting of refers to compositions, methods, and respective components thereof as described herein, which are exclusive of any element not recited in that description of the embodiment.

Although a few variations have been described in detail above, other modifications or additions are possible.

In the descriptions above and in the paragraphs, phrases such as "at least one of' or "one or more of' may occur followed by a conjunctive list of elements or features. The term "and/or" may also occur in a list of two or more elements or features. Unless otherwise implicitly or explicitly contradicted by the context in which it is used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar Interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, B and C together, or A and B and C together." In addition, use of the term "based on," above and in the paragraphs is intended to mean, "based at least in part on," such that an unrecited feature or element is also permissible.

The subject matter described herein can be embodied in Systems, apparatus, methods, and/or articles depending on the desired configuration. The implementations set forth in the foregoing description do not represent all implementations consistent with the subject matter described herein. Instead, they are merely some examples consistent with aspects related to the described subject matter. Although a few variations have been described in detail above, other modifications or additions are possible. In particular, further features and/or variations can be provided in addition to those set forth herein. For example, the implementations described above can be directed to various combinations and sub-combinations of the disclosed features and/or combinations and sub-combinations of several further features disclosed above. In addition, the logic flows depicted in the accompanying figures and/or described herein do not necessarily require the particular order shown, or sequential Order, to achieve desirable results. Other implementations may be within the scope of the following Claims.

## Claims

1. A system (300) comprising:
a detector (310) configured to acquire a plurality of images of a tissue;
a digital storage device (322) configured to store the plurality of images;
an image recognition system configured to identify a target within the tissue based on an initial image of the plurality of images;
an electromagnetic radiation, EMR, system (320) configured to generate an EMR beam having a wavelength that is absorbable by the tissue; and
a controller (314) configured to control the EMR system to fractionally direct the EMR beam to impact and ablate a first portion of a surface of the tissue containing the target, wherein the first portion comprises a first plurality of non-adjacent locations on the surface of the tissue,
wherein the controller (314) is further configured to (i) compare one or more subsequent images of the plurality of images and the initial image to generate comparison data, (ii) determine a remaining portion of the target based on the comparison data, and (iii) control the EMR system to fractionally direct the EMR beam to impact and ablate a second portion of the surface of the tissue containing the remaining portion of the target, wherein the second portion does not overlap with the first portion.

2. The system (300) of claim 1, wherein the wavelength of the EMR beam is within a set of ranges which are at least one of (i) 1300 - 3500 nm and (ii) 9 - 11 µm.

3. The system (300) of claim 1, wherein the target comprises at least one target comprising at least one of (i) a sebaceous gland, (ii) a eccrine gland, (iii) a hair follicle and (iv) a tattoo.

4. The system (300) of claim 1, wherein the image recognition system further comprises at least one of a neural network, an artificial intelligence, a clinical decision support system and a machine vision system.

5. The system (300) of claim 1, wherein the detector (310) is configured to facilitate a time period to elapse between the acquiring of the initial image and the one or more subsequent images which is longer than 12 hours.

6. The system (300) of claim 1, wherein the digital storage device (322) comprises at least one of a network storage device, a flash drive, a USB drive, a hard disk drive and a memory device, and wherein the digital storage device (322) is configured to store an electronic health record.

7. The system (300) of claim 1, wherein the image recognition system comprises at least one of an edge detection module, a corner detection module and a blob detection module.

8. The system (300) of claim 1, wherein the detector (310) comprises at least one of a camera, an ultrasound transducer, a photoacoustic imaging system, an optical coherence tomography system, an optical coherence elastography system, a coherent anti-stokes Raman spectroscopy imaging system, a two-photon imaging system, second harmonic generation imaging system, a phase conjugate imaging system, a hyperspectral imaging system, a low-power carbon-dioxide laser imaging system, X-ray backscatter imaging system, a millimeter wave imaging system, a magnetic resonance imaging system, a high-frequency ultrasound imaging system, a photodiode, an ultrasound transducer array, a fluoroscope, a surface profilometer, an infrared imaging system and a confocal microscope.

9. The system (300) of claim 1, further comprising a structured light source, wherein the detector (310) comprises at least one of a fringe projection profilometry configuration, a structure light profilometry configuration, a laser triangulation profilometry configuration and a stereovision measurement configuration.

10. A non-therapeutic and non-surgical method of treatment of skin for cosmetic purposes comprising:
acquiring a plurality of images of a tissue;
storing the plurality of images to or in a digital storage device (322);
generating, using an electromagnetic radiation, EMR, system (320), an EMR beam having a wavelength that is absorbable by the tissue;
identifying, by an image recognition system, a target within the tissue based upon the an initial image of the plurality of images; and controlling, by a the controller (314), the EMR system to fractionally direct the EMR beam to impact and ablate a first portion of a surface of the tissue containing the target, wherein the first portion comprises a first plurality of non-adjacent locations on the surface of the tissue, comparing one or more subsequent images of the plurality of images and the initial image to generate comparison data;
determining a remaining portion of the target based on the comparison data; and
controlling the EMR system to fractionally direct the EMR beam to impact and ablate a second portion of the surface of the tissue containing the remaining portion of the target, wherein the second portion does not overlap with the first portion.

11. A computer-accessible medium having computer software thereon, wherein, when the computer software is executed by a computer processor, the computer processor is configured to perform procedures comprising:
controlling an image acquisition system to acquire a plurality of images of a tissue; storing the plurality of images to a digital storage device (322);
controlling an electromagnetic radiation, EMR, system (320) to generate an EMR beam having a wavelength that is absorbable by the tissue;
identifying a target within the tissue based upon an initial image of the plurality of images; and controlling the EMR system to fractionally direct the EMR beam to impact and ablate a first portion of a surface of the tissue containing the target, wherein the first portion comprises a first plurality of non-adjacent locations on the surface of the tissue, comparing one or more subsequent images of the plurality of images and the initial image to generate comparison data;
determining a remaining portion of the target based on the comparison data; and
controlling the EMR system to fractionally direct the EMR beam to impact and ablate a second portion of the surface of the tissue containing the remaining portion of the target, wherein the second portion does not overlap with the first portion.

## Patentansprüche

1. System (300), umfassend:
einen Detektor (310), der konfiguriert ist, um eine Vielzahl von Bildern eines Gewebes zu erfassen;
eine digitale Speichervorrichtung (322), die konfiguriert ist, um die Vielzahl von Bildern zu speichern;
ein Bilderkennungssystem, das konfiguriert ist, um ein Ziel innerhalb des Gewebes basierend auf einem Anfangsbild der Vielzahl von Bildern zu identifizieren;
ein elektromagnetisches Strahlungs- ,EMR, System (320), das konfiguriert ist, um einen EMR-Strahl mit einer Wellenlänge zu erzeugen, die von dem Gewebe absorbierbar ist; und
eine Steuerung (314), die konfiguriert ist, um das EMR-System zu steuern, um den EMR-Strahl fraktioniert zu lenken, um auf einen ersten Abschnitt einer Oberfläche des Gewebes, der das Ziel enthält, aufzutreffen und diesen abzutragen, wobei der erste Abschnitt eine erste Vielzahl von nicht benachbarten Stellen auf der Oberfläche des Gewebes umfasst,
wobei die Steuerung (314) ferner konfiguriert ist, um (i) ein oder mehrere nachfolgende Bilder der Vielzahl von Bildern und das Anfangsbild zu vergleichen, um Vergleichsdaten zu erzeugen, (ii) einen verbleibenden Abschnitt des Ziels basierend auf den Vergleichsdaten zu bestimmen, und (iii) das EMR-System zu steuern, um den EMR-Strahl fraktioniert zu lenken, um auf einen zweiten Abschnitt der Oberfläche des Gewebes, der den verbleibenden Abschnitt des Ziels enthält, aufzutreffen und diesen abzutragen, wobei der zweite Abschnitt nicht mit dem ersten Abschnitt überlappt.

2. System (300) nach Anspruch 1, wobei die Wellenlänge des EMR-Strahls innerhalb eines Satzes von Bereichen liegt, die mindestens eines von (i) 1300 - 3500 nm und (ii) 9 - 11 µm sind.

3. System (300) nach Anspruch 1, wobei das Ziel mindestens ein Ziel umfassend mindestens eines von (i) einer Talgdrüse, (ii) einer ekkrinen Drüse, (iii) einem Haarfollikel und (iv) einer Tätowierung, umfasst.

4. System (300) nach Anspruch 1, wobei das Bilderkennungssystem ferner mindestens eines von einem neuronalen Netzwerk, einer künstlichen Intelligenz, einem klinischen Entscheidungsunterstützungssystem und einem maschinellen Bildverarbeitungssystem umfasst.

5. System (300) nach Anspruch 1, wobei der Detektor (310) konfiguriert ist, um zu ermöglichen, dass ein Zeitintervall zwischen dem Erfassen des Anfangsbildes und dem einen oder den mehreren nachfolgenden Bildern verstreicht, der länger als 12 Stunden ist.

6. System (300) nach Anspruch 1, wobei die digitale Speichervorrichtung (322) mindestens eines von einer Netzwerkspeichervorrichtung, einem Flash-Laufwerk, einem USB-Laufwerk, einem Festplattenlaufwerk und einer Speichervorrichtung umfasst, und wobei die digitale Speichervorrichtung (322) konfiguriert ist, um eine elektronische Gesundheitsakte zu speichern.

7. System (300) nach Anspruch 1, wobei das Bilderkennungssystem mindestens eines von einem Kantendetektionsmodul, einem Eckendetektionsmodul und einem Blob-Detektionsmodul umfasst.

8. System (300) nach Anspruch 1, wobei der Detektor (310) mindestens eines von einer Kamera, einem Ultraschallwandler, einem photoakustischen Bildgebungssystem, einem optischen Kohärenztomographiesystem, einem optischen Kohärenzelastografiesystem, einem kohärenten Anti-Stokes-Raman-Spektroskopie-Bildgebungssystem, einem Zwei-Photonen-Bildgebungssystem, einem Bildgebungssystem der zweiten harmonischen Generation, einem Phasenkonjugations-Bildgebungssystem, einem hyperspektralen Bildgebungssystem, einem Kohlenstoffdioxid-Laserbildgebungssystem mit geringer Leistung, einem Röntgenrückstreubildgebungssystem, einem Millimeterwellen-Bildgebungssystem, einem Magnetresonanzbildgebungssystem, einem Hochfrequenz-Ultraschallbildgebungssystem, einer Fotodiode, einer Ultraschallwandleranordnung, einem Fluoroskop, einem Oberflächenprofilometer, einem Infrarotbildgebungssystem und einem Konfokalmikroskop, umfasst.

9. System (300) nach Anspruch 1, ferner umfassend eine strukturierte Lichtquelle, wobei der Detektor (310) mindestens eines von einer Streifenprojektions-Profilometrie-Konfiguration, einer Strukturlicht-Profilometrie-Konfiguration, einer Lasertriangulations-Profilometrie-Konfiguration und einer Stereovisions-Messkonfiguration umfasst.

10. Nicht-therapeutisches und nicht-chirurgisches Verfahren zur Behandlung von Haut für kosmetische Zwecke, umfassend:
Erfassen einer Vielzahl von Bildern eines Gewebes;
Speichern der Vielzahl von Bildern auf oder in einer digitalen Speichervorrichtung (322);
Erzeugen, unter Verwendung eines elektromagnetischen Strahlungs, EMR, - Systems (320), eines EMR-Strahls mit einer Wellenlänge, die von dem Gewebe absorbierbar ist;
Identifizieren, durch ein Bilderkennungssystem, eines Ziels innerhalb des Gewebes basierend auf dem Anfangsbild der Vielzahl von Bildern; und
Steuern, durch eine Steuerung (314), des EMR-Systems, um den EMR-Strahl fraktioniert zu lenken, um auf einen ersten Abschnitt einer Oberfläche des Gewebes, der das Ziel enthält, aufzutreffen und diesen abzutragen, wobei der erste Abschnitt eine erste Vielzahl von nicht benachbarten Stellen auf der Oberfläche des Gewebes umfasst,Vergleichen eines oder mehrerer nachfolgender Bilder der Vielzahl von Bildern und des Anfangsbildes, um Vergleichsdaten zu erzeugen;
Bestimmen eines verbleibenden Abschnitts des Ziels basierend auf den Vergleichsdaten; und
Steuern des EMR-Systems, um den EMR-Strahl fraktioniert zu lenken, um auf einen zweiten Abschnitt der Oberfläche des Gewebes, der den verbleibenden Abschnitt des Ziels enthält, aufzutreffen und diesen abzutragen, wobei der zweite Abschnitt nicht mit dem ersten Abschnitt überlappt.

11. Computerzugängliches Medium mit Computersoftware darauf, wobei, wenn die Computersoftware durch einen Computerprozessor ausgeführt wird, der Computerprozessor konfiguriert ist, um Vorgänge durchzuführen, umfassend:
Steuern eines Bilderfassungssystems, um eine Vielzahl von Bildern eines Gewebes zu erfassen; Speichern der Vielzahl von Bildern auf einer digitalen Speichervorrichtung (322);
Steuern eines elektromagnetischen Strahlungs, EMR, -Systems (320), um einen EMR-Strahl mit einer Wellenlänge zu erzeugen, der von dem Gewebe absorbierbar ist;
Identifizieren eines Ziels innerhalb des Gewebes basierend auf einem Anfangsbild der Vielzahl von Bildern; und
Steuern des EMR-Systems, um den EMR-Strahl fraktioniert zu lenken, um auf einen ersten Abschnitt einer Oberfläche des Gewebes, der das Ziel enthält, aufzutreffen und diesen abzutragen, wobei der erste Abschnitt eine erste Vielzahl von nicht benachbarten Stellen auf der Oberfläche des Gewebes umfasst,Vergleichen eines oder mehrerer nachfolgender Bilder der Vielzahl von Bildern und des Anfangsbildes, um Vergleichsdaten zu erzeugen;
Bestimmen eines verbleibenden Abschnitts des Ziels basierend auf den Vergleichsdaten; und
Steuern des EMR-Systems, um den EMR-Strahl fraktioniert zu lenken, um auf einen zweiten Abschnitt der Oberfläche des Gewebes, der den verbleibenden Abschnitt des Ziels enthält, aufzutreffen und diesen abzutragen, wobei der zweite Abschnitt nicht mit dem ersten Abschnitt überlappt.

## Revendications

1. Système (300), comprenant:
un détecteur (310) configuré pour acquérir une pluralité d'images d'un tissu;
un dispositif de stockage numérique (322) configuré pour stocker la pluralité d'images;
un système de reconnaissance d'image configuré pour identifier une cible à l'intérieur du tissu sur la base d'une image initiale de la pluralité d'images;
un système de rayonnement électromagnétique, EMR, (320) configuré pour générer un faisceau EMR ayant une longueur d'onde qui est absorbable par le tissu; et
un dispositif de commande (314) configuré pour commander le système EMR pour diriger de manière fractionnée le faisceau EMR pour frapper et ablater une première partie d'une surface du tissu contenant la cible, la première partie comprenant une première pluralité d'emplacements non adjacents sur la surface du tissu,
dans lequel le dispositif de commande (314) est en outre configuré pour (i) comparer une ou plusieurs images ultérieures de la pluralité d'images et l'image initiale pour générer des données de comparaison, (ii) déterminer une partie restante de la cible sur la base des données de comparaison, et (iii) commander le système EMR pour diriger de manière fractionnée le faisceau EMR pour frapper et ablater une seconde partie de la surface du tissu contenant la partie restante de la cible, la seconde partie ne chevauchant pas la première partie.

2. Système (300) selon la revendication 1, dans lequel la longueur d'onde du faisceau EMR est dans un ensemble de plages qui sont au moins l'une parmi (i) 1300-3500 nm et (ii) 9-11 µm.

3. Système (300) selon la revendication 1, dans lequel la cible comprend au moins une cible comprenant au moins l'un parmi (i) une glande sébacée, (ii) une glande eccrine, (iii) un follicule pileux, et (iv) un tatouage.

4. Système (300) selon la revendication 1, dans lequel le système de reconnaissance d'image comprend en outre au moins l'un parmi un réseau neuronal, une intelligence artificielle, un système d'aide à la décision clinique, et un système de traitement d'image machine.

5. Système (300) selon la revendication 1, dans lequel le détecteur (310) est configuré pour permettre à un intervalle de temps entre l'acquisition de l'image initiale et les une ou plusieurs images ultérieures de s'écouler qui est supérieur à 12 heures.

6. Système (300) selon la revendication 1, dans lequel le dispositif de stockage numérique (322) comprend au moins l'un parmi un dispositif de stockage de réseau, un lecteur flash, un lecteur USB, un lecteur de disque dur, et un dispositif de stockage, et dans lequel le dispositif de stockage numérique (322) est configuré pour stocker un dossier de santé électronique.

7. Système (300) selon la revendication 1, dans lequel le système de reconnaissance d'image comprend au moins l'un parmi un module de détection de bord, un module de détection de coin, et un module de détection de blob.

8. Système (300) selon la revendication 1, dans lequel le détecteur (310) comprend au moins l'un parmi une caméra, un transducteur ultrasonore, un système d'imagerie photoacoustique, un système de tomographie par cohérence optique, un système d'élastographie par cohérence optique, un système d'imagerie par spectroscopie Raman anti-Stokes cohérente, un système d'imagerie à deux photons, un système d'imagerie de deuxième génération harmonique, un système d'imagerie par conjugaison de phase, un système d'imagerie hyperspectrale, un système d'imagerie laser à dioxyde de carbone à faible puissance, un système d'imagerie par rétrodiffusion de rayons X, un système d'imagerie à ondes millimétriques, un système d'imagerie par résonance magnétique, un système d'imagerie ultrasonore à haute fréquence, une photodiode, un réseau de transducteurs ultrasonores, un fluoroscope, un profilomètre de surface, un système d'imagerie infrarouge, et un microscope confocal.

9. Système (300) selon la revendication 1, comprenant en outre une source de lumière structurée, dans lequel le détecteur (310) comprend au moins l'une parmi une configuration de profilométrie par projection de franges, une configuration de profilométrie par lumière structurée, une configuration de profilométrie par triangulation laser, et une configuration de mesure de stéréovision.

10. Procédé non thérapeutique et non chirurgical de traitement de la peau à des fins cosmétiques, comprenant:
l'acquisition d'une pluralité d'images d'un tissu;
le stockage de la pluralité d'images sur ou dans un dispositif de stockage numérique (322);
la génération, à l'aide d'un système de rayonnement électromagnétique, EMR, (320), d'un faisceau EMR ayant une longueur d'onde qui est absorbable par le tissu;
l'identification, par un système de reconnaissance d'image, d'une cible à l'intérieur du tissu sur la base de l'image initiale de la pluralité d'images; et
la commande, par un dispositif de commande (314), du système EMR pour diriger de manière fractionnée le faisceau EMR pour frapper et ablater une première partie d'une surface du tissu contenant la cible, la première partie comprenant une première pluralité d'emplacements non adjacents sur la surface du tissu,la comparaison d'une ou plusieurs images ultérieures de la pluralité d'images et de l'image initiale pour générer des données de comparaison;
la détermination d'une partie restante de la cible sur la base des données de comparaison; et
la commande du système EMR pour diriger de manière fractionnée le faisceau EMR pour frapper et ablater une seconde partie de la surface du tissu contenant la partie restante de la cible, la seconde partie ne chevauchant pas la première partie.

11. Support accessible par ordinateur ayant un logiciel informatique sur celui-ci, dans lequel, lorsque le logiciel informatique est exécuté par un processeur informatique, le processeur informatique est configuré pour effectuer des opérations comprenant:
la commande d'un système d'acquisition d'images pour acquérir une pluralité d'images d'un tissu; le stockage de la pluralité d'images sur un dispositif de stockage numérique (322);
la commande d'un système de rayonnement électromagnétique, EMR, (320) pour générer un faisceau EMR ayant une longueur d'onde qui est absorbable par le tissu;
l'identification d'une cible à l'intérieur du tissu sur la base d'une image initiale de la pluralité d'images; et
la commande du système EMR pour diriger de manière fractionnée le faisceau EMR pour frapper et ablater une première partie d'une surface du tissu contenant la cible, la première partie comprenant une première pluralité d'emplacements non adjacents sur la surface du tissu,la comparaison d'une ou plusieurs images ultérieures de la pluralité d'images et de l'image initiale pour générer des données de comparaison;
la détermination d'une partie restante de la cible sur la base des données de comparaison; et
la commande du système EMR pour diriger de manière fractionnée le faisceau EMR pour frapper et ablater une seconde partie de la surface du tissu contenant la partie restante de la cible, la seconde partie ne chevauchant pas la première partie.
